# EUROPEAN PATENT APPLICATION

(11) **EP 4 494 640 A1**
(43) Date of publication of application: **22.01.2025**
(21) Application number: 23771129.6
(22) Date of filing: 17.03.2023
(51) Int. Cl.: A61K 31/416, A61K 31/4439, A61P 43/00, C07D 231/56, A61P 35/00, A61P 29/00, A61P 37/00, C07D 401/06, A23L 33/10

(54) **NOVEL HETEROCYCLIC INHIBITOR FOR HISTONE DEACETYLASE, AND PHARMACEUTICAL COMPOSITION COMPRISING SAME**

(30) Priority: 17.03.2022 KR 20220033173; 17.03.2022 KR 20220033174; 16.03.2023 KR 20230034303
(71) Applicant: Sookmyung Women's University Industry-Academic Cooperation Foundation, Seoul 04310 (KR)
(72) Inventor: JEON, Raok, Seoul 04310 (KR); MISHRA, Chandra Bhushan, Seoul 04310 (KR); KIM, Jisoo, Seoul 04310 (KR)
(74) Representative: Mewburn Ellis LLP
(86) International application number: PCT/KR2023/003562
(87) International publication number: WO 2023/177250

(57) **Abstract**

The present invention relates to a novel heterocyclic inhibitor for histone deacetylase (HDAC), a novel therapeutic agent for HDAC-associated diseases, and a pharmaceutical composition comprising same. More specifically, a compound according to the present invention exhibits a more selective and effective inhibitory activity with respect to HDAC6 even from among HDACs, and thus can be effectively used as a therapeutic agent for various HDAC6-associated diseases such as cancer, inflammatory diseases, autoimmune diseases, fibrotic diseases and degenerative diseases.

## Description

### Technical Field

The present disclosure relates to a novel heterocyclic compound having a histone deacetylase inhibitory ability and a medical use thereof, particularly to an HDAC6 inhibitor which is more selective and effective on HDAC6 among histone deacetylases (HDACs), and treatment of histone deacetylase-associated diseases.

### Background Art

A histone deacetylase (HDAC) plays an important role in gene expression and differentiation as well as maintenance of cellular homeostasis by promoting a process of removing acetyl groups from acetylated 3-aminolysine residues on histone and non-histone proteins. HDACs consist of four classes and 18 subtypes, classified into Class I (HDAC1, 2, 3, and 8), Class II (HDAC4, 5, 7, 9, 6, and 10), Class III (SIRT1-7), and Class IV (HDAC11), respectively. HDACs, which belong to Classes I, II, and IV, are Zn²⁺-dependent enzymes, while Class III HDACs are characterized as nicotinamide adenine dinucleotide (NAD)-dependent enzymes.

Of these HDACs, HDAC6 consists of 1215 amino acid residues including CD1 and CD2 catalytic domains in the N-terminal and central regions, respectively. The C-terminus has a zinc-finger ubiquitin binding domain that facilitates the interaction between ubiquitin proteasomes and the aggresome pathway. In addition to deacetylation of histones, HDAC6 induces deacetylation using non-histone proteins as substrates such as α-tubulin, cortactin, tau, peroxiredoxin, and heat shock protein (HSP90).

Numerous reports suggest that HDAC6 is associated with various physiological processes such as cell migration, autophagy, endocytosis, protein transport, apoptosis, and degradation, and owing to these important physiological functions, HDAC6 is becoming an effective therapeutic target for various human diseases such as cancer, inflammation, and immune disorders.

To date, various types of HDAC inhibitors have been researched and developed, of which five types of FDA-approved HDAC-inhibiting anticancer drugs are in use. However, since these drugs are non-selective HDAC inhibitors, there is a need to develop selective inhibitors which target characteristic isoforms while having reduced side effects thereby. Due to the large similarities among HDAC subtypes, it is not easy to discover subtype selective inhibitors, but there is a high demand for the development of more selective, effective HDAC6 inhibitors.

### Disclosure of the Invention

### Technical Goals

An object of the present disclosure is to provide a novel HDAC6 inhibitor.

Another object of the present disclosure is to provide a method of inhibiting HDAC6 using the novel HDAC6 inhibitor.

Another object of the present disclosure is to provide a therapeutic agent for a HDAC6-associated disease through the discovery of a novel HDAC6 inhibitor.

Another object of the present disclosure is to provide a method of treating a HDAC6-associated disease, including administering a novel HDAC6 inhibitor to a subject in need of treatment for a HDAC6-associated disease.

### Technical Solutions

The present disclosure provides a compound selected from a compound represented by the following Chemical Formula 1 or a pharmaceutically acceptable salt thereof:

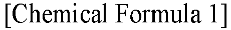

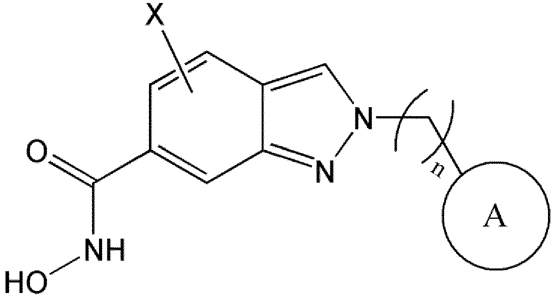

in the Chemical Formula 1,
X is selected from hydrogen, fluorine, chlorine, or bromine,
A is selected from substituted or unsubstituted phenyl, naphthyl, or a heterocyclic compound,
the substitution is carried out with one or more substituents selected from (C1~C10)alkyl, (C1~C10)alkoxy, halo, difluoromethyl, trifluoromethyl, hydroxy, cyano, nitro, phenyl, benzyloxy, or phenoxy,
the heterocyclic compound is a heteroaryl compound with 5 or 6 rings, and
n is an integer of 1 to 3.

In addition, the present disclosure provides a pharmaceutical composition for treating or preventing a histone deacetylase 6 (HDAC6)-associated disease, including a compound selected from the compound represented by Chemical Formula 1 or a pharmaceutically acceptable salt thereof.

In addition, the present disclosure provides a health functional food for ameliorating or preventing a histone deacetylase 6 (HDAC6)-associated disease, including a compound selected from the compound represented by Chemical Formula 1 or a pharmaceutically acceptable salt thereof.

In addition, the present disclosure provides a composition for inhibiting histone deacetylase 6 (HDAC6), including a compound selected from the compound represented by Chemical Formula 1 or a pharmaceutically acceptable salt thereof.

In addition, the present disclosure provides a method of inhibiting histone deacetylase 6 (HDAC6) including treating a compound selected from the compound represented by Chemical Formula 1 or a pharmaceutically acceptable salt thereof.

In addition, the present disclosure provides a method of treating a histone deacetylase 6 (HDAC6)-associated disease including administrating a compound selected from the compound represented by Chemical Formula 1 or a pharmaceutically acceptable salt thereof to a subject suffering from the histone deacetylase 6 (HDAC6)-associated disease.

### Advantageous Effects

The present disclosure relates to a novel heterocyclic histone deacetylase inhibitor, and specifically, by providing an HDAC6 inhibitor which is more selective and effective on HDAC6 among histone deacetylases (HDACs), it is possible to develop various disease treatments for HDAC6-associated diseases such as cancer diseases, inflammatory diseases, autoimmune diseases, fibrotic diseases, and degenerative diseases.

### Best Mode for Carrying Out the Invention

Hereinafter, the present disclosure will be described in detail.

With an endeavor to develop a more effective HDAC6 inhibitor, the present inventors completed the present disclosure by synthesizing a novel indazole-based HDAC6 inhibitor and identifying an outstanding HDAC6 inhibitory activity.

Hereby, the present disclosure provides a compound selected from a compound represented by the following Chemical Formula 1 or a pharmaceutically acceptable salt thereof:

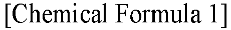

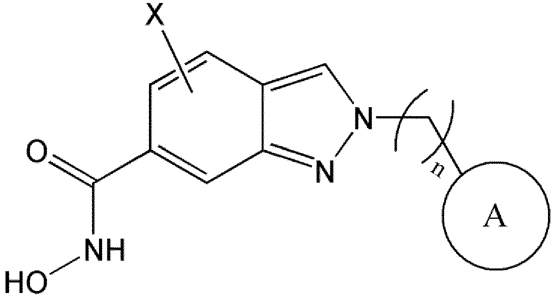

in the Chemical Formula 1,
X may be selected from hydrogen, fluorine, chlorine, or bromine,
A may be selected from substituted or unsubstituted phenyl, naphthyl, or a heterocyclic compound,
the substitution may be carried out with one or more substituents selected from (C1∼C10)alkyl, (C1∼C10)alkoxy, halo, difluoromethyl, trifluoromethyl, hydroxy, cyano, nitro, phenyl, benzyloxy, or phenoxy,
the heterocyclic compound may be a heteroaryl compound with 5 or 6 rings, and
n may be an integer of 1 to 3.

The heterocyclic compound may be selected from the group consisting of, but is not limited to, thiophenes, furans, pyrazoles, pyridines, pyrans, oxazines, thiazines, pyrimidines, and piperazines.

Preferably, the compound may be one represented by the following Chemical Formula 1-1:

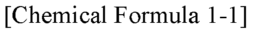

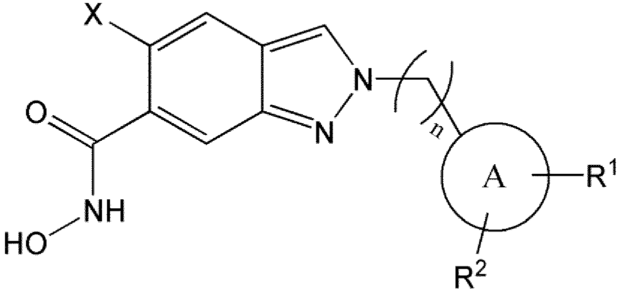

in the Chemical Formula 1-1,
X may be hydrogen or fluorine,
A may be selected from phenyl, naphthyl, or pyrimidyl,
R¹ and R² may be the same or different and selected from hydrogen, (C1~C4)alkyl, (C1~C4)alkoxy, halo, difluoromethyl, trifluoromethyl, phenyl, benzyloxy, or phenoxy, and
n may be an integer of 1 to 3.

More preferably, in the compound, i) A may be selected from phenyl, naphthyl, or pyrimidyl, R¹ may be selected from hydrogen, (C1~C4)alkyl, (C1~C4)alkoxy, halo, difluoromethyl, trifluoromethyl, phenyl, benzyloxy, or phenoxy, R² may be selected from hydrogen, (C1~C4)alkoxy, or halo, and n may be an integer of 1 to 2.

In an example embodiment, the compound may be selected from the group consisting of 2-benzyl-2H-indazole-6-carboxylic acid hydroxyamide (Compound 50); 2-(4-methoxybenzyl)-2H-indazole-6-carboxylic acid hydroxyamide (Compound 51); 2-(4-methylbenzyl)-2H-indazole-6-carboxylic acid hydroxyamide (Compound 52); 2-(4-fluorobenzyl)-2H-indazole-6-carboxylic acid hydroxyamide (Compound 53); 2-(4-chlorobenzyl)-2H-indazole-6-carboxylic acid hydroxyamide (Compound 54); 2-(4-bromobenzyl)-2H-indazole-6-carboxylic acid hydroxyamide (Compound 55); 2-(3-trifluoromethoxybenzyl)-2H-indazole-6-carboxylic acid hydroxyamide (Compound 56); 2-(4-trifluoromethoxybenzyl)-2H-indazole-6-carboxylic acid hydroxyamide (Compound 57); 2-naphthalen-2-ylmethyl-2H-indazole-6-carboxylic acid hydroxyamide (Compound 58); 2-phenethyl-2H-indazole-6-carboxylic acid hydroxyamide (Compound 59); 2-(2-4-methoxyphenylethyl)-2H-indazole-6-carboxylic acid hydroxyamide (Compound 60); 2-(3,5-difluorobenzyl)-2H-indazole-6-carboxylic acid hydroxyamide (Compound 61); 2-(4-difluoromethoxybenzyl)-2H-indazole-6-carboxylic acid hydroxyamide (Compound 62); 2-biphenyl-4-ylmethyl-2H-indazole-6-carboxylic acid hydroxyamide (Compound 63); 2-(4-isopropylbenzyl)-2H-indazole-6-carboxylic acid hydroxyamide (Compound 64); 2-(2-(4-fluorophenyl)ethyl)-2H-indazole-6-carboxylic acid hydroxyamide (Compound 65); 2-(3-fluorobenzyl)-2H-indazole-6-carboxylic acid hydroxyamide (Compound 66); 2-(3-chlorobenzyl)-2H-indazole-6-carboxylic acid hydroxyamide (Compound 67); 2-(3-trifluoromethylbenzyl)-2H-indazole-6-carboxylic acid hydroxyamide (Compound 68); 2-(4-trifluoromethylbenzyl)-2H-indazole-6-carboxylic acid hydroxyamide (Compound 69); 2-(3-methoxybenzyl)-2H-indazole-6-carboxylic acid hydroxyamide (Compound 70); 2-(3-benzyloxybenzyl)-2H-indazole-6-carboxylic acid hydroxyamide (Compound 71); 2-(3,5-dimethoxybenzyl)-2H-indazole-6-carboxylic acid hydroxyamide (Compound 72); 2-(3-phenoxybenzyl)-2H-indazole-6-carboxylic acid hydroxyamide (Compound 73); 2-pyridin-3-ylmethyl-2H-indazole-6-carboxylic acid hydroxyamide (Compound 76); 2-benzyl-5-fluoro-2H-indazole-6-carboxylic acid hydroxyamide (Compound 88); 2-(3-chlorobenzyl)-5-fluoro-2H-indazole-6-carboxylic acid hydroxyamide (Compound 89); and 2-(3-methoxybenzyl)-5-fluoro-2H-indazole-6-carboxylic acid hydroxyamide (Compound 90).

The compound according to the present disclosure is capable of inhibiting histone deacetylase 6 (HDAC6) more selectively.

The term 'pharmaceutically acceptable salt' as used herein may be used in either form of a pharmaceutically acceptable basic salt or acidic salt. The basic salt may be used in any form of organic or inorganic salts, such as sodium salts, potassium salts, calcium salts, lithium salts, magnesium salts, cesium salts and aminium salts, ammonium salts, triethylaminium salts, and pyridinium salts, but is not limited thereto.

In addition, as the acidic salt, an acid additive salt which is formed by a free acid is useful. An inorganic acid and an organic acid may be used as the free acid, hydrochloric acid, bromic acid, sulfurous acid, sulfurous acid, or phosphoric acid may be used as the inorganic acid, and citric acid, acetic acid, maleic acid, fumaric acid, gluconic acid, methanesulfonic acid, benzenesulfonic acid, camphorsulfonic acid, oxalic acid, malonic acid, glutaric acid, acetic acid, glycolic acid, succinic acid, tartaric acid, 4-toluenesulfonic acid, galacturonic acid, embonic acid, glutamic acid, citric acid, and aspartic acid may be used as the organic acid. Preferably, hydrochloric acid may be used as the inorganic acid, and methanesulfonic acid as the organic acid.

In addition, the compound according to the present disclosure may include not only pharmaceutically acceptable salts, but also all salts, hydrates, and solvents that may be prepared by conventional methods.

The additive salt according to the present disclosure may be prepared by conventional methods, for example, it may be prepared by dissolving the compound in a water-miscible organic solvent, e.g., acetone, methanol, ethanol, or acetonitrile and then precipitating or crystallizing through addition of an excess of organic bases or an aqueous solution of bases of inorganic bases. Alternatively, preparation may be performed by evaporating and drying solvents or excess bases from this mixture to obtain the additive salts, or by suction-filtering the precipitated salts.

In addition, the present disclosure provides a pharmaceutical composition for treating or preventing a histone deacetylase 6 (HDAC6)-associated disease, including a compound selected from the compound represented by Chemical Formula 1 or a pharmaceutically acceptable salt thereof.

In addition, the present disclosure provides a method of treating a histone deacetylase 6 (HDAC6)-associated disease, including administrating a compound selected from the compound represented by Chemical Formula 1 or a pharmaceutically acceptable salt thereof to a subject suffering from the histone deacetylase 6 (HDAC6)-associated disease.

The histone deacetylase 6 (HDAC6)-associated disease may be selected from the group consisting of cancer diseases, inflammatory diseases, autoimmune diseases, fibrotic diseases, and degenerative diseases.

The cancer disease may be selected from the group consisting of, but is not limited to, colon cancer, lung cancer, kidney cancer, bladder cancer, liver cancer, thymic cancer, ovarian cancer, cervical cancer, breast cancer, colorectal cancer, stomach cancer, pancreatic cancer, colon cancer, peritoneal metastatic cancer, skin cancer, bladder cancer, prostate cancer, osteosarcoma, fibrous tumor, brain tumor, acute lymphocytic leukemia, acute myeloid leukemia, lymphoma, and neuroblastoma.

The above inflammatory disease or autoimmune disease may be selected from the group consisting of, but is not limited to, osteoarthritis, rheumatoid arthritis, dermatitis, allergies, atopy, asthma, psoriasis, conjunctivitis, rhinitis, otitis media, sore throat, tonsillitis, pneumonia, gastric ulcer, gastritis, Crohn's disease, inflammatory bowel disease, lupus, hepatitis, cystitis, interstitial cystitis, nephritis, sjogren's syndrome, multiple sclerosis, Hashimoto thyroiditis, polymyositis, scleroderma, Addison disease, vitiligo, pernicious anemia, cystic fibrosis, graft-versus-host disease, graft rejection disease, autoimmune diabetes, diabetic retinopathy, ischemia-reperfusion injury, post-angioplasty restenosis, chronic obstructive pulmonary disease (COPD), Graves disease, and acute and chronic inflammatory diseases.

The fibrotic disease may be selected from the group consisting of, but is not limited to, pulmonary fibrosis, renal fibrosis, cardiac fibrosis, liver fibrosis, scleroderma, skeletal muscle fibrosis, and diabetic fibrosis.

In addition, the liver fibrosis may include, but is not limited to, non-alcoholic steatohepatitis (NASH) or non-alcoholic fatty liver disease (NAFLD).

The degenerative disease may be, but is not limited to, a neurodegenerative disease selected from the group consisting of cerebral infarction, stroke, memory loss, memory impairment, dementia, forgetfulness, Parkinson's disease, Alzheimer's disease, Pick's disease, Creutzfeldt-Jakob disease, Huntington's disease, and Lou Gehrig's disease.

The pharmaceutical composition may be provided in one or more formulations selected from the group consisting of gels, emulsions, injections, acids, granules, aerosols, pastes, transdermal absorbents, and patches in accordance with conventional methods, but is not limited thereto.

In another example embodiment of the present disclosure, the pharmaceutical composition may further include one or more additives selected from the group consisting of suitable carriers, excipients, disintegrating agents, sweeteners, coating agents, swelling agents, lubricants, glydents, flavoring agents, antioxidants, buffers, bacteriostatics, diluents, dispersing agents, surfactants, binders, and antifrictions that are commonly used in the manufacture of pharmaceutical compositions.

Specifically, for carriers, excipients, and diluents, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil may be used, and solid preparations for oral administration include tablets, pills, acids, granules, and capsules, wherein these solid preparations may be prepared by mixing, in the composition, at least one or more excipients, for example, starch, calcium carbonate, sucrose or lactose, and gelatin. In addition to simple excipients, lubricants such as magnesium stearate and talc may also be used. For oral liquid preparations, suspensions, liquids, emulsions, and syrups may be used, and various excipients, such as humectants, sweeteners, fragrances, and preservatives, may be included in addition to the simple diluents that are commonly used, such as water and liquid paraffin. Preparations for parenteral administration include sterile aqueous solutions, non-aqueous solvents, suspensions, emulsions, lyophilized agents, and suppositories. For non-aqueous solvents and suspensions, propylene glycol, polyethylene glycol, vegetable oils such as olive oil, and injectable esters such as ethyl oleate may be used. For a base of suppositories, witepsol, macrogol, tween 61, cacao butter, laurin butter, and glycerogelatin may be used.

The pharmaceutical composition may be administered to a subject in a conventional manner through the intravenous, intra-arterial, intraperitoneal, intramuscular, intra-arterial, intraperitoneal, intrasternal, percutaneous, nasal, inhaled, topical, rectal, oral, intraocular, or intradermal routes.

The preferred dosage of the compound may vary depending on the condition and weight of the subject, the type and severity of disease, the drug form, and the route and duration of administration, and it may be appropriately selected by those skilled in the art. According to an example embodiment of the present disclosure, the daily dosage may be, but is not limited to, 0.01 to 200 mg/kg, specifically 0.1 to 200 mg/kg, and more specifically 0.1 to 100 mg/kg. The administration may be performed once a day or in several divided doses, but the scope of the present disclosure is not limited thereby.

In the present disclosure, the 'subject' may be a mammal including human, but is not limited to these examples.

In addition, the present disclosure provides a health functional food for ameliorating or preventing a histone deacetylase 6 (HDAC6)-associated disease, including a compound selected from the compound represented by Chemical Formula 1 or a pharmaceutically acceptable salt thereof.

The term "health functional food" as used herein refers to food manufactured and processed with raw materials or ingredients having useful functionality for the human body in accordance with the Health Functional Food Act, and the term "functionality" as used herein refers to the intake to derive effectiveness in health care such as physiological actions or regulation of nutrients for the structure and function of the human body.

The health functional food may include conventional food additives, and the suitability as the "food additive" is determined by the standards and criteria related to corresponding items according to the general rules and general test methods of Korean Food Additives Codex approved by the Ministry of Food and Drug Safety, unless otherwise stipulated.

The items listed in the "Korean Food Additives Codex" may include, for example, chemically synthesized compounds such as ketones, glycine, potassium citrate, nicotinic acid, and cinnamic acid, natural additives such as persimmon color, licorice extracts, crystallized cellulose, kaoliang color, and guar gum, and mixed preparations such as sodium L-glutamate preparations, noodle-added alkali agents, preservative agents, and tar color agents.

The effective dose of the active ingredient contained in the health functional food may be used in accordance with the effective dose of the therapeutic agent, but in the case of long-term intake for the purpose of health and hygiene or for the purpose of health control, it may be below the above range, but it is certain that the active ingredient may be used in an amount above the range since there is no problem in terms of safety.

There is no specific restriction on the types of health functional foods, such as meat, sausages, bread, chocolate, candy, snacks, confectionery, pizza, ramen, other noodles, chewing gum, dairy products including ice cream, various soups, beverages, tea, drinks, alcoholic beverages, and vitamin complexes.

In addition, the present disclosure provides a composition for inhibiting histone deacetylase 6 (HDAC6), including a compound selected from the compound represented by Chemical Formula 1 or a pharmaceutically acceptable salt thereof.

In addition, the present disclosure provides a method of inhibiting histone deacetylase 6 (HDAC6), including treating a compound selected from the compound represented by Chemical Formula 1 or a pharmaceutically acceptable salt thereof.

The description on the compound is the same as those set forth above, such that repeated descriptions are omitted.

The composition may be utilized as a pharmaceutical composition, health functional food, and reagent composition.

### Modes for Carrying Out the Invention

Hereinafter, the present disclosure will be described in detail through examples. These examples are merely intended to describe the present disclosure in more detail, and it will be apparent to those with ordinary skill in the art to which the present disclosure pertains that the scope of the present disclosure is not limited to these examples according to the gist of the present disclosure.

### <Example> Synthesis of indazole derivatives as HDAC6 inhibitors

As an HDAC6 inhibitor, indazole derivatives were synthesized via typical processes according to Scheme 1, Scheme 2, or Scheme 3 below. The specific synthesis processes are then described in detail for each compound. 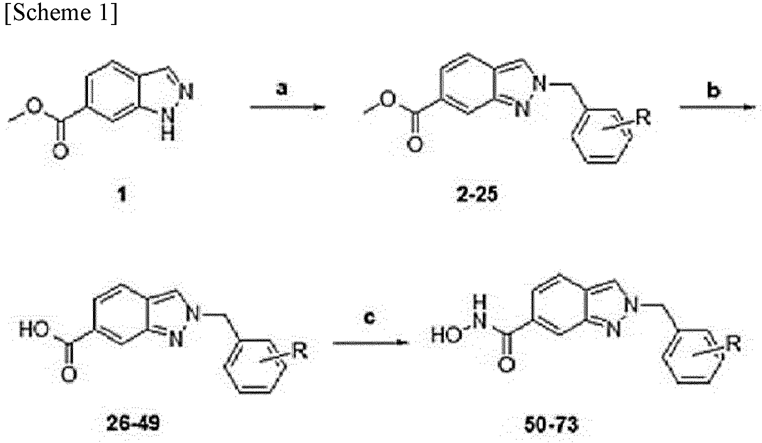
(a) Alkyl bromide/chloride, Cs₂CO₃, DMF, 100°C, 4-8 hours;
(b) 2N NaOH, MeOH, room temperature (rt), 6-12 hours;
(c) NH₂OH, BOP, DIPEA, DMSO, room temperature (rt), 6-8 hours.

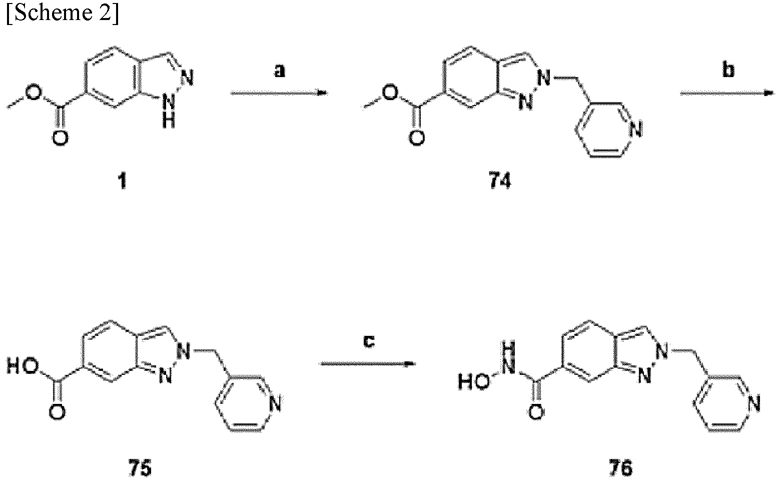

(a) Alkyl bromide/chloride, Cs₂CO₃, DMF, 100°C, 4-8 hours;
(b) 2N NaOH, MeOH, room temperature (rt), 6-12 hours;
(c) NH₂OH, BOP, DIPEA, DMSO, room temperature (rt), 6-8 hours. 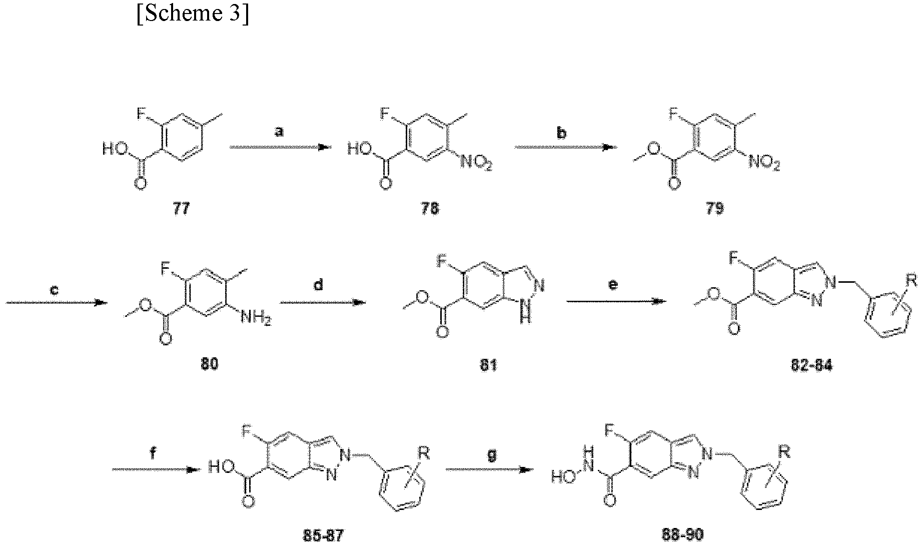
   (a) H₂SO₄, HNO₃, 0°C, 3 hours;
   (b) H₂SO₄, MeOH, 65°C, 20 hours;
   (c) Pd/C, H₂, EtOAc, room temperature (rt), 2 hours;
   (d) KOAc, AcOH, isoamyl nitrate, CHCl₃, room temperature (rt), 3 hours;
   (e) Alkyl bromide/chloride, Cs₂CO₃, DMF, room temperature (rt), 2 hours;
   (f) 2NNaOH, MeOH, THF, room temperature (rt), 2 hours;
   (g) NH₂OH, BOP, DIPEA, DMSO, room temperature (rt), 6-8 hours.

### 1. Synthesis of N2-substituted 2H-indazole-6-carboxylic acid methyl ester compounds (Compound 2 to Compound 25, Compound 74)

In DMF (15 ml), a mixture of indazole (1; 5.02 mmol), alkyl bromide/chloride (5.52 mmol), and Cs₂CO₃ (5.52 mmol) was reacted by heating at 100°C for 4-8 hours. After completion of the reaction, the reaction mixture was added to water, and the crude product was extracted with ethyl acetate. N2-substituted indazole derivatives (2-25, 74) were separated respectively via column chromatography using hexane:ethyl acetate (80:20) as an eluate.
1) 2-Benzyl-2H-indazole-6-carboxylic acid methyl ester [Compound 2]
   White solid; Yield of 45%, ¹H NMR (500 MHz, DMSO-d₆) δ 8.62 (s, 1H), 8.30 (s, 1H), 7.84 (d, J = 8.7 Hz, 1H), 7.57 (d, J = 8.7 Hz, 1H), 7.42 - 7.29 (m, 5H), 5.72 (s, 2H), 3.88 (s, 3H).
2) 2-(4-Methoxybenzyl)-2H-indazole-5-carboxylic acid methyl ester [Compound 3]
   White solid; Yield of 42%, ¹H NMR (500 MHz, DMSO) δ 8.56 (s, 1H), 8.29 (s, 1H), 7.82 (d, J = 8.8 Hz, 1H), 7.56 (d, J = 8.8 Hz, 1H), 7.35 (d, J = 8.6 Hz, 2H), 6.93 (d, J = 8.6 Hz, 2H), 5.63 (s, 2H), 3.88 (s, 3H), 3.73 (s, 3H).
3) 2-(4-Methylbenzyl)-2H-indazole-6-carboxylic acid methyl ester [Compound 4]
   White solid; Yield of 40%, ¹H NMR (500 MHz, DMSO-d₆) δ 8.58 (s, 1H), 8.29 (s, 1H), 7.83 (d, J = 8.7 Hz, 1H), 7.57 (d, J = 8.7 Hz, 1H), 7.27 (d, J = 7.6 Hz, 2H), 7.17 (d, J = 7.6 Hz, 2H), 5.66 (s, 2H), 3.88 (s, 3H), 2.28 (s, 3H).
4) 2-(4-Fluorobenzyl)-2H-indazole-6-carboxylic acid methyl ester [Compound 5]
   White solid; Yield of 38%, ¹H NMR (500 MHz, DMSO-d₆) δ 8.62 (s, 1H), 8.29 (s, 1H), 7.84 (d, J = 8.7 Hz, 1H), 7.57 (d, J = 8.7 Hz, 1H), 7.49 - 7.40 (m, 2H), 7.23 - 7.17 (m, 2H), 5.71 (s, 2H), 3.88 (s, 3H).
5) 2-(4-Chlorobenzyl)-2H-indazole-6-carboxylic acid methyl ester [Compound 6]
   White solid; Yield of 41%, ¹H NMR (500 MHz, DMSO-d₆) δ 8.63 (s, 1H), 8.29 (s, 1H), 7.84 (d, J = 9.5 Hz, 1H), 7.58 (d, J = 9.5 Hz, 1H), 7.44 (d, J = 8.5 Hz, 2H), 7.38 (d, J = 8.5 Hz, 2H), 5.73 (s, 2H), 3.88 (s, 3H).
6) 2-(4-Bromobenzyl)-2H-indazole-5-carboxylic acid methyl ester [Compound 7]
   White solid; Yield of 39%, 1H NMR (500 MHz, MeOD-d₄) δ 8.41 (s, 1H), 8.39 (s, 1H), 7.80 (d, J = 8.7 Hz, 1H), 7.69 (d, J = 8.7 Hz, 1H), 7.54 (d, J = 7.5 Hz, 2H), 7.27 (d, J = 7.5 Hz, 2H), 5.68 (s, 2H), 3.95 (s, 3H).
7) 2-(3-Trifluoromethoxybenzyl)-2H-indazole-5-carboxylic acid methyl ester [Compound 8]
8) 2-(4-Trifluoromethoxybenzyl)-2H-indazole-5-carboxylic acid methyl ester [Compound 9]
   White solid; Yield of 40%, ¹H NMR (500 MHz, DMSO-d₆) δ 8.65 (s, 1H), 8.31 (s, 1H), 7.86 - 7.27 (m, 6H), 5.77 (s, 2H), 3.88 (s, 3H).
9) 2-Naphthalen-2-ylmethyl-2H-indazole-6-carboxylic acid methyl ester [Compound 10]
   White solid; Yield of 45%, ¹H NMR (500 MHz, DMSO-d₆) δ 8.68 (s, 1H), 8.30 (s, 1H), 7.93 - 7.89 (m, 4H), 7.85 (d, J = 8.8 Hz, 1H), 7.61 - 7.47 (m, 4H), 3.88 (s, 3H).
10) 2-Phenethyl-2H-indazole-5-carboxylic acid methyl ester [Compound 11]
   White solid; Yield of 41%, ¹H NMR (500 MHz, DMSO-d₆) δ 8.39 (s, 1H), 8.31 (s, 1H), 7.79 (d, J = 9.4 Hz, 1H), 7.55 (d, J = 9.4 Hz, 1H), 7.25 - 7.18 (m, 5H), 4.75 (t, J = 7.3 Hz, 2H), 3.89 (s, 3H), 3.29 (t, J = 7.3 Hz, 2H).
11) 2-(2-4-Methoxyphenylethyl)-2H-indazole-5-carboxylic acid methyl ester [Compound 12]
   White solid; Yield of 42%, ¹H NMR (500 MHz, DMSO-d₆) δ 8.37 (s, 1H), 8.33 (s, 1H), 7.78 (d, J = 8.7 Hz, 1H), 7.56 (d, J = 8.7 Hz, 1H), 7.09 (d, J = 8.6 Hz, 2H), 6.81 (d, J = 8.6 Hz, 2H), 4.69 (t, J = 7.3 Hz, 2H), 3.88 (s, 3H), 3.69 (s, 3H), 3.22 (t, J = 7.3 Hz, 2H).
12) 2-(3,5-Difluorobenzyl)-2H-indazole-5-carboxylic acid methyl ester [Compound 13]
   White solid; Yield of 38%, ¹H NMR (500 MHz, DMSO-d₆) δ 8.66 (s, 1H), 8.31 (s, 1H), 7.86 (d, J = 9.4 Hz, 1H), 7.59 (d, J = 9.4 Hz, 1H), 7.21 (tt, J = 8.9 Hz, 2.3Hz, 1H), 7.11 - 7.05 (m, 2H), 5.76 (s, 2H), 3.88 (s, 3H).
13) 2-(4-Difluoromethoxybenzyl)-2H-indazole-5-carboxylic acid methyl ester [Compound 14]
   White solid; Yield of 39%, ¹H NMR (500 MHz, DMSO-d₆) δ 8.63 (s, 1H), 8.29 (s, 1H), 7.84 (d, J = 8.8 Hz, 1H), 7.57 (d, J = 8.8 Hz, 1H), 7.44 (d, J = 8.6 Hz, 2H), 7.36 (t, J = 74.0, 1H), 7.18 (d, J = 8.6), 5.72 (s, 2H), 3.88 (s, 3H).
14) 2-Biphenyl-4-ylmethyl-2H-indazole-5-carboxylic acid methyl ester [Compound 15]
   White solid; Yield of 42%, ¹H NMR (500 MHz, DMSO-d₆) δ 8.66 (s, 1H), 8.31 (s, 1H), 7.85 (d, J = 8.7 Hz, 1H), 7.71 - 7.61 (m, 4H), 7.58 (d, J = 8.7 Hz, 1H), 7.47 - 7.43 (m, 4H), 7.37 (t, J = 7.3 Hz, 1H), 5.77 (s, 2H), 3.88 (s, 3H).
15) 2-(4-Isopropylbenzyl)-2H-indazole-6-carboxylic acid methyl ester [Compound 16]
   White solid; Yield of 36%, ¹H NMR (500 MHz, CDCl₃) δ 8.52 (s, 1H), 7.90 (s, 1H), 7.70 (dd, J = 8.8, 1.3 Hz, 1H), 7.64 (dd, J = 8.8, 0.8 Hz, 1H), 7.25 - 7.20 (m, 4H), 5.59 (s, 2H), 3.95 (s, 3H), 2.90 (hept, J = 6.9 Hz, 1H), 1.23 (d, J = 6.9 Hz, 6H).
16) 2-(2-(4-Fluorophenyl)ethyl)-2H-indazole-6-carboxylic acid methyl ester [Compound 17]
   White solid; Yield of 20%, ¹H NMR (500 MHz, CDCl₃) δ 8.52 (s, 1H), 7.70 (dd, J = 8.6, 1.1 Hz, 2H), 7.62 (dd, J = 8.6, 0.6 Hz, 1H), 7.06 - 6.97 (m, 2H), 6.97 - 6.87 (m, 2H), 4.63 (t, J = 7.1 Hz, 2H), 3.96 (s, 3H), 3.31 (t, J = 7.1 Hz, 2H).
17) 2-(3-Fluorobenzyl)-2H-indazole-6-carboxylic acid methyl ester [Compound 18] [142] White solid; Yield of 39%, ¹H NMR (500 MHz, CDCl₃) δ 8.52 (d, J = 1.3 Hz, 1H), 7.94 (s, 1H), 7.70 (dd, J = 8.8, 1.4 Hz, 1H), 7.64 (d, J = 8.8 Hz, 1H), 7.30 (q, J = 8.0 Hz, 1H), 7.11 - 6.97 (m, 2H), 6.95 (d, J = 9.5 Hz, 1H), 5.58 (s, 2H), 3.93 (s, 3H); ¹³C NMR (125 MHz, CDCl₃) δ 167.5, 163.0, 148.3, 137.8, 130.6, 128.1, 124.2, 123.5, 123.3, 121.6, 121.4, 120.3, 115.5, 114.93, 57.2, 52.2.
18) 2-(3-Chlorobenzyl)-2H-indazole-6-carboxylic acid methyl ester [Compound 19]
   White solid; Yield of 39%, ¹H NMR (500 MHz, CDCl₃) δ 8.52 (s, 1H), 7.95 (s, 1H), 7.72 (dd, J = 8.8, 1.3 Hz, 1H), 7.67 (dd, J = 8.8, 0.7 Hz, 1H), 7.34 - 7.28 (m, 3H), 7.17 (d, J = 7.0 Hz, 1H), 5.60 (s, 2H), 3.95 (s, 3H).
19) 2-(3-Trifluoromethylbenzyl)-2H-indazole-6-carboxylic acid methyl ester [Compound 20]
   White solid; Yield of 36%, ¹H NMR (500 MHz, CDCl₃) δ 8.52 (q, J = 1.1 Hz, 1H), 7.97 (d, J = 1.1 Hz, 1H), 7.73 (dd, J = 8.7, 1.4 Hz, 1H), 7.67 (dd, J = 8.7, 1.0 Hz, 1H), 7.60 (d, J = 7.4 Hz, 1H), 7.59 - 7.57 (m, 1H), 7.52 - 7.41 (m, 2H), 5.68 (s, 2H), 3.95 (s, 3H); ¹³C NMR (125 MHz, CDCl₃) δ 167.5, 148.4, 136.4, 131.3, 131.3, 129.6, 128.2, 125.5, 124.7, 123.8, 124.3, 123.2, 121.8, 121.5, 120.2, 57.3, 52.2.
20) 2-(4-Trifluoromethylbenzyl)-2H-indazole-6-carboxylic acid methyl ester [Compound 21]
   White solid; Yield of 37%, ¹H NMR (500 MHz, CDCl₃) δ 8.52 (d, J = 1.1 Hz, 1H), 7.97 (d, J = 1.1 Hz, 1H), 7.72 (dd, J = 8.8, 1.4 Hz, 1H), 7.66 (dd, J = 8.8, 1.0 Hz, 1H), 7.60 (d, J = 8.1 Hz, 2H), 7.37 (d, J = 8.1 Hz, 2H), 5.67 (s, 2H), 3.94 (s, 3H).
21) 2-(3-Methoxybenzyl)-2H-indazole-6-carboxylic acid methyl ester [Compound 22]
   White solid; Yield of 39%, ¹H NMR (500 MHz, CDCl₃) δ 8.52 (d, J = 0.9 Hz, 1H), 7.91 (d, J = 0.9 Hz, 1H), 7.70 (dd, J = 8.8, 1.3 Hz, 1H), 7.64 (dd, J = 8.8, 0.6 Hz, 1H), 7.27 (t, J = 7.9 Hz, 1H), 6.91 - 6.85 (m, 2H), 6.83 (d, J = 1.7 Hz, 1H), 5.58 (s, 2H), 3.94 (s, 3H), 3.76 (s, 3H); ¹³C NMR (125 MHz, CDCl₃) δ 167.6, 160.1, 148.1, 136.7, 130.1, 127.9, 124.2, 123.1, 121.5, 121.4, 120.3, 120.2, 114.0, 113.8, 57.9, 55.3, 52.2.
22) 2-(3-Benzyloxybenzyl)-2H-indazole-6-carboxylic acid methyl ester [Compound 23]
   White solid; Yield of 42%, ¹H NMR (500 MHz, CDCl₃) δ 8.52 (d, J = 1.1 Hz, 1H), 7.89 (d, J = 1.1 Hz, 1H), 7.71 (dd, J = 8.8, 1.4 Hz, 1H), 7.65 (dd, J = 8.8, 0.9 Hz, 1H), 7.39 - 7.27 (m, 6H), 6.96 - 6.94 (m, 1H), 6.92 - 6.84 (m, 2H), 5.59 (s, 2H), 5.02 (s, 2H), 3.95 (s, 3H); ¹³C NMR (125 MHz, CDCl₃) δ 167.6, 159.2, 148.1, 136.8, 136.6, 130.1, 128.6, 128.1, 127.9, 127.5, 124.2, 123.2, 121.5, 121.4, 120.6, 120.3, 115.0, 114.7, 70.0, 57.8, 52.2.
23) 2-(3,5-Dimethoxybenzyl)-2H-indazole-6-carboxylic acid methyl ester [Compound 24]
   White solid; Yield of 40%, ¹H NMR (500 MHz, CDCl₃) δ 8.51 (d, J = 1.1 Hz, 1H), 7.92 (d, J = 1.1 Hz, 1H), 7.69 (dt, J = 8.8, 1.4 Hz, 1H), 7.65 - 7.63 (m, 1H), 6.46 - 6.37 (m, 3H), 5.54 - 5.52 (m, 2H), 3.94 (q, J = 1.4 Hz, 3H), 3.74 (q, J = 1.4 Hz, 6H); ¹³C NMR (125 MHz, CDCl₃) δ 167.6, 161.3, 148.1, 137.4, 127.9, 124.2, 123.2, 121.5, 121.4, 120.3, 106.2, 100.3, 58.0, 55.4, 52.2.
24) 2-(3-Phenoxybenzyl)-2H-indazole-6-carboxylic acid methyl ester [Compound 25]
   White solid; Yield of 32%, ¹H NMR (500 MHz, CDCl₃) δ 8.51 (d, J = 1.1 Hz, 1H), 7.92 (d, J = 1.1 Hz, 1H), 7.70 (dd, J = 8.8, 1.4 Hz, 1H), 7.64 (dd, J = 8.8, 0.9 Hz, 1H), 7.31 - 7.27 (m, 3H), 7.14 - 7.06 (m, 1H), 7.02 - 6.96 (m, 3H), 6.96 - 6.90 (m, 2H), 5.57 (s, 2H), 3.93 (s, 3H); ¹³C NMR (125 MHz, CDCl₃) δ 167.5, 158.0, 156.6, 148.2, 137.3, 130.4, 129.9, 128.0, 124.2, 123.7, 123.2, 122.5, 121.5, 121.4, 120.2, 119.2, 118.5, 118.2, 57.6, 52.2.
25) 2-Pyridin-3-ylmethyl-2H-indazole-6-carboxylic acid methyl ester [Compound 74]
   White solid; Yield of 51%, ¹H NMR (500 MHz, CDCl₃) δ 8.72 - 8.55 (m, 2H), 8.51 (d, J = 1.0 Hz, 1H), 7.98 (d, J = 0.6 Hz, 1H), 7.72 (dd, J = 8.8, 1.3 Hz, 1H), 7.67 (dd, J = 8.8, 0.8 Hz, 1H), 7.61 (d, J = 7.9 Hz, 1H), 7.33 - 7.27 (m, 1H), 5.65 (s, 2H), 3.95 (s, 3H).

### 2. Synthesis of N2-substituted 2H-indazole-6-carboxylic acid (Compound 26 to Compound 49, Compound 75)

N2-substituted 2H-indazole-6-carboxylic acid methyl ester (Compounds 2 to 25 and 74, 1 mmol) was dissolved in methanol (5-10 ml), and 2N NaOH solution (1-2 ml) was slowly added. After stirring the reaction mixture for 6-12 hours at room temperature, it was placed in water and neutralized with 2N HCl, and then the precipitate was filtered and dried to obtain carboxylic acid derivatives (Compounds 26-49, Compound 75).
1) 2-Benzyl-2H-indazole-5-carboxylic acid [Compound 26]
   White solid; Yield of 75%, ¹H NMR (500 MHz, DMSO-d₆) δ 12.88 (s, 1H), 8.60 (s, 1H), 8.27 (s, 1H), 7.80 (d, J = 8.8 Hz, 1H), 7.57 (d, J = 8.8 Hz, 1H), 7.41 - 7.28 (m, 5H), 5.71 (s, 2H).
2) 2-(4-Methoxybenzyl)-2H-indazole-5-carboxylic acid [Compound 27]
   White solid; Yield of 64%, ¹H NMR (500 MHz, DMSO-d₆) δ 12.87 (s, 1H), 8.54 (s, 1H), 8.26 (s, 1H), 7.79 (d, J = 8.7 Hz, 1H), 7.56 (d, J = 8.7 Hz, 1H), 7.35 (d, J = 8.2 Hz, 2H), 6.93 (d, J = 8.2 Hz, 2H), 5.62 (s, 2H), 3.73 (s, 3H).
3) 2-(4-Methylbenzyl)-2H-indazole-5-carboxylic acid [Compound 28]
   White solid; Yield of 69%, ¹H NMR (500 MHz, DMSO-d₆) δ 12.88 (s, 1H), 8.55 (s, 1H), 8.26 (s, 1H), 7.79 (d, J = 8.7 Hz, 1H), 7.56 (d, J = 8.7 Hz, 1H), 7.26 (d, J = 7.9 Hz, 2H), 7.17 (d, J = 7.9 Hz, 2H), 5.65 (s, 2H), 2.27 (s, 3H).
4) 2-(4-Fluorobenzyl)-2H-indazole-5-carboxylic acid [Compound 29]
   White solid; Yield of 77%, ¹H NMR (500 MHz, DMSO-d₆) δ 12.89 (s, 1H), 8.60 (s, 1H), 8.27 (s, 1H), 7.80 (d, J = 8.5 Hz, 1H), 7.57 (d, J = 8.5 Hz, 1H), 7.45 - 7.41 (m, 2H), 7.20 (t, J = 8.9 Hz, 2H), 5.70 (s, 2H).
5) 2-(4-Chlorobenzyl)-2H-indazole-5-carboxylic acid [Compound 30]
   White solid; Yield of 71%, ¹H NMR (500 MHz, DMSO-d₆) δ 12.90 (s, 1H), 8.61 (s, 1H), 8.26 (s, 1H), 7.81 (d, J = 9.4 Hz, 1H), 7.57 (d, J = 9.4 Hz, 1H), 7.44 (d, J = 8.5 Hz, 2H), 7.37 (d, J = 8.5 Hz, 2H), 5.72 (s, 2H).
6) 2-(4-Bromobenzyl)-2H-indazole-5-carboxylic acid [Compound 31]
   White solid; Yield of 73%, 1H NMR (500 MHz, DMSO-d₆) δ 12.88 (s, 1H), 8.60 (s, 1H), 8.26 (s, 1H), 7.81 (d, J = 8.5 Hz, 1H), 7.58 - 7.56 (m, 3H), 7.31 (d, J = 8.4 Hz, 2H), 5.70 (s, 2H).
7) 2-(3-Trifluoromethoxybenzyl)-2H-indazole-5-carboxylic acid [Compound 32]
   White solid; Yield of 79%, ¹H NMR (500 MHz, DMSO-d₆) δ 12.90 (s, 1H), 8.44 (s, 1H), 8.41 (s, 1H), 7.80 (d, J = 9.5 Hz, 1H), 7.71 (d, J = 9.5 Hz, 1H), 7.48 (t, J = 7.8 Hz, 1H), 7.32 (d, J = 7.8 Hz, 1H), 7.29 - 7.25 (m, 2H), 5.76 (s, 3H).
8) 2-(4-Trifluoromethoxybenzyl)-2H-indazole-5-carboxylic acid [Compound 33]
   White solid; Yield of 69%, ¹H NMR (500 MHz, DMSO-d₆) δ 12.90 (s, 1H), 8.64 (s, 1H), 8.27 (s, 1H), 7.81 (d, J = 9.4 Hz, 1H), 7.57 (d, J = 9.4 Hz, 1H), 7.48 (d, J = 8.7 Hz, 2H), 7.37 (d, J = 8.7 Hz, 2H), 5.76 (s, 2H).
9) 2-Naphthalen-2-ylmethyl-2H-indazole-5-carboxylic acid [Compound 34]
   White solid; Yield of 71%, ¹H NMR (500 MHz, DMSO-d₆) δ 12.89 (s, 1H), 8.66 (s, 1H), 8.28 (s, 1H), 7.92 - 7.90 (m, 4H), 7.82 (d, J = 8.7 Hz, 1H), 7.64 - 7.45 (m, 4H), 5.88 (s, 2H).
10) 2-Phenethyl-2H-indazole-5-carboxylic acid [Compound 35]
   White solid; Yield of 79%, ¹H NMR (500 MHz, DMSO-d₆) δ 12.88 (s, 1H), 8.37 (s, 1H), 8.29 (s, 1H), 7.75 (d, J = 9.2 Hz, 1H), 7.55 (d, J = 9.2 Hz, 1H), 7.30 - 7.22 (m, 2H), 7.21 - 7.19 (m, 3H), 4.74 (t, J = 7.3 Hz, 2H), 3.29 (t, J = 7.3 Hz, 2H).
11) 2-[2-(4-Methoxyphenylethyl)]-2H-indazole-5-carboxylic acid [Compound 36]
   White solid; Yield of 82%, ¹H NMR (500 MHz, DMSO-d₆) δ 12.90 (s, 1H), 8.36 (s, 1H), 8.29 (s, 1H), 7.75 (d, J = 8.7 Hz, 1H), 7.55 (d, J = 8.7 Hz, 1H), 7.09 (d, J = 8.6 Hz, 2H), 6.81 (d, J = 8.6 Hz, 2H), 4.68 (t, J = 7.3 Hz, 2H), 3.69 (s, 3H), 3.22 (t, J = 7.3 Hz, 2H).
12) 2-(3,5-Difluorobenzyl)-2H-indazole-5-carboxylic acid [Compound 37]
   White solid; Yield of 68%, ¹H NMR (500 MHz, DMSO-d₆) δ 12.96 (s, 1H), 8.65 (s, 1H), 8.28 (s, 1H), 7.83 (d, J = 8.7 Hz, 1H), 7.58 (d, J = 8.7 Hz, 1H), 7.23 (tt, J = 8.9 Hz, 2.3Hz, 1H), 7.09 - 7.05 (m, 2H), 5.75 (s, 2H).
13) 2-(4-Difluoromethoxybenzyl)-2H-indazole-5-carboxylic acid [Compound 38]
   White solid; Yield of 60%, ¹H NMR (500 MH DMSO-d₆) δ 12.89 (s, 1H), 8.60 (s, 1H), 8.27 (s, 1H), 7.81 (d, J = 8.7 Hz, 1H), 7.57 (d, J = 8.7 Hz, 1H), 7.44 (d, J = 8.6 Hz, 2H), 7.22 (t, J = 74.0), 7.20 (d, J = 8.6 Hz, 2H), 5.71 (s, 2H).
14) 2-Biphenyl-4-ylmethyl-2H-indazole-5-carboxylic acid [Compound 39]
   White solid; Yield of 71%, ¹H NMR (500 MHz, DMSO-d₆) δ 12.90 (s, 1H), 8.64 (s, 1H), 8.29 (s, 1H), 7.82 (d, J = 8.7 Hz, 1H), 7.67 - 7.62 (m, 4H), 7.58 (d, J = 9.9 Hz, 1H), 7.48 - 7.44 (m, 4H), 7.36 (t, J = 7.4 Hz, 1H), 5.76 (s, 2H).
15) 2-(4-Isopropylbenzyl)-2H-indazole-6-carboxylic acid [Compound 40]
   White solid; Yield of 96%, ¹H NMR (500 MHz, DMSO-d₆) δ 12.85 (s, 1H), 8.56 (s, 1H), 8.24 (s, 1H), 7.77 (d, J = 8.7 Hz, 1H), 7.55 (d, J = 8.7 Hz, 1H), 7.28 (d, J = 8.1 Hz, 2H), 7.22 (d, J = 8.1 Hz, 2H), 5.64 (s, 2H), 2.90 - 2.81 (m, 1H), 1.16 (d, J = 6.9 Hz, 6H).
16) 2-[2-(4-Fluorophenylethyl)]-2H-indazole-6-carboxylic acid [Compound 41]
   White solid; Yield of 61%, ¹H NMR (500 MHz, DMSO-d₆) δ 12.88 (s, 1H), 8.35 (s, 1H), 8.27 (s, 1H), 7.75 (d, J = 8.7 Hz, 1H), 7.55 (d, J = 8.7 Hz, 1H), 7.21 (dd, J = 8.3, 5.7 Hz, 2H), 7.07 (t, J = 8.3 Hz, 2H), 4.72 (t, J = 7.2 Hz, 2H), 3.28 (t, J = 7.2 Hz, 2H).
17) 2-(3-Fluorobenzyl)-2H-indazole-6-carboxylic acid [Compound 42]
   White solid; Yield of 83%,¹H NMR (500 MHz, DMSO-d₆) δ 12.90 (s, 1H), 8.62 (s, 1H), 8.27 (s, 1H), 7.81 (d, J = 8.7 Hz, 1H), 7.57 (d, J = 8.7 Hz, 1H), 7.40 (q, J = 7.5 Hz, 1H), 7.27 - 7.05 (m, 3H), 5.73 (s, 2H); ¹³C NMR (125 MHz, DMSO-d₆) δ 168.2, 162.6, 148.0, 139.8, 131.2, 128.8, 125.4, 124.5, 124.0, 121.4, 121.2, 120.5, 115.4, 115.2, 56.4.
18) 2-(3-Chlorobenzyl)-2H-indazole-6-carboxylic acid [Compound 43]
   White solid; Yield of 92%, ¹H NMR (500 MHz, DMSO-d₆) δ 12.90 (s, 1H), 8.63 (s, 1H), 8.26 (s, 1H), 7.81 (d, J = 8.8 Hz, 1H), 7.56 (dd, J = 8.8, 1.2 Hz, 1H), 7.42 (s, 1H), 7.41 - 7.33 (m, 2H), 7.33 - 7.25 (m, 1H), 5.72 (s, 2H); ¹³C NMR (125 MHz, DMSO-d₆) δ 168.2, 148.0, 139.4, 133.6, 131.1, 128.7, 128.5, 128.3, 127.2, 125.4, 124.0, 121.5, 121.2, 120.5, 98.7, 56.3.
19) 2-(3-Trifluoromethylbenzyl)-2H-indazole-6-carboxylic acid [Compound 44]
   White solid; Yield of 97%, ¹H NMR (500 MHz, DMSO-d₆) δ 12.90 (s, 1H), 8.66 (s, 1H), 8.26 (s, 1H), 7.82 (d, J = 8.7 Hz, 1H), 7.77 (s, 1H), 7.72 - 7.68 (m, 1H), 7.64 - 7.53 (m, 3H), 5.83 (s, 2H); ¹³C NMR (125 MHz, DMSO-d₆) δ 168.2, 148.1, 141.3, 138.5, 132.7, 130.3, 129.7, 128.8, 125.6, 125.3, 125.1, 124.0, 121.5, 121.2, 120.5, 56.3.
20) 2-(4-Trifluoromethylbenzyl)-2H-indazole-6-carboxylic acid [Compound 45]
   White solid; Yield of 93%, ¹H NMR (500 MHz, DMSO-d₆) δ 12.91 (s, 1H), 8.65 (s, 1H), 8.26 (s, 1H), 7.82 (d, J = 8.8 Hz, 1H), 7.73 (d, J = 8.2 Hz, 2H), 7.57 (dd, J = 8.8, 1.2 Hz, 1H), 7.51 (d, J= 8.1 Hz, 2H), 5.83 (s, 2H); ¹³C NMR (125 MHz, DMSO-d₆) δ 168.2, 148.1, 141.8, 141.8, 129.1, 128.8, 126.0, 125.6, 124.0, 121.6, 121.5, 121.2, 120.5, 56.4.
21) 2-(3-Methoxybenzyl)-2H-indazole-6-carboxylic acid [Compound 46]
   White solid; Yield of 67%, ¹H NMR (500 MHz, DMSO-d₆) δ 12.86 (s, 1H), 8.57 (s, 1H), 8.26 (s, 1H), 7.79 (d, J = 8.6 Hz, 1H), 7.56 (d, J = 8.6 Hz, 1H), 7.27 (t, J = 7.6 Hz, 1H), 6.98 - 6.83 (m, 3H), 5.66 (s, 2H), 3.73 (s, 3H); ¹³C NMR (125 MHz, DMSO-d₆) δ 159.9, 147.9, 138.5, 130.3, 125.2, 125.1, 124.0, 121.4, 121.1, 121.1, 120.6, 120.5, 117.5, 114.3, 113.8, 57.0, 55.6.
22) 2-(3-Benzyloxybenzyl)-2H-indazole-6-carboxylic acid [Compound 47]
   White solid; Yield of 98%, ¹H NMR (500 MHz, DMSO-d₆) δ 12.90 (s, 1H), 8.57 (s, 1H), 8.27 (s, 1H), 7.80 (d, J = 8.5 Hz, 1H), 7.57 (d, J = 8.5 Hz, 1H), 7.45 - 7.18 (m, 6H), 7.01 (s, 1H), 6.93 (dd, J = 24.9, 7.7 Hz, 2H), 5.66 (s, 2H), 5.06 (s, 2H); ¹³C NMR (125 MHz, DMSO-d₆) δ 168.3, 158.9, 147.9, 138.5, 137.3, 130.3, 128.9, 128.3, 128.2, 125.2, 124.0, 121.4, 121.1, 120.8, 120.6, 115.2, 114.6, 69.7, 57.0.
23) 2-(3,5-Dimethoxybenzyl)-2H-indazole-6-carboxylic acid [Compound 48]
   White solid; Yield of 87%, ¹H NMR (500 MHz, DMSO-d₆) δ 12.90 (s, 1H), 8.57 (s, 1H), 8.27 (s, 1H), 7.79 (d, J = 8.7 Hz, 1H), 7.62 - 7.51 (m, 1H), 6.51 (d, J = 2.1 Hz, 2H), 6.45 (t, J = 2.1 Hz, 1H), 5.61 (s, 2H), 3.70 (s, 6H); ¹³C NMR (125 MHz, DMSO-d₆) δ 168.3, 161.1, 147.9, 139.1, 128.6, 125.2, 124.0, 121.4, 121.1, 120.6, 106.7, 99.9, 57.1, 55.7.
24) 3-Phenoxybenzyl)-2H-indazole-6-carboxylic acid [Compound 49]
   White solid; Yield of 99%, ¹H NMR (500 MHz, DMSO-d₆) δ 12.91 (s, 1H), 8.59 (s, 1H), 8.25 (s, 1H), 7.79 (d, J = 8.7 Hz, 1H), 7.56 (d, J = 8.7 Hz, 1H), 7.43 - 7.30 (m, 3H), 7.14 (t, J = 7.4 Hz, 1H), 7.07 (d, J = 7.7 Hz, 1H), 7.05 - 6.94 (m, 3H), 6.92 (dd, J = 8.1, 2.0 Hz, 1H), 5.70 (s, 2H); ¹³C NMR (125 MHz, DMSO-d₆) δ 168.3, 157.3, 156.7, 148.0, 139.3, 130.8, 130.6, 125.3, 124.2, 124.0, 123.3, 121.4, 121.2, 121.1, 120.5, 119.3, 118.5, 118.3, 56.6.
25) 2-Pyridin-3-ylmethyl-2H-indazole-6-carboxylic acid [Compound 75]
   White solid; Yield of 80%, ¹H NMR (500 MHz, DMSO-d₆) δ 12.90 (s, 1H), 9.02 (s, 1H), 8.88 (d, J = 5.4 Hz, 1H), 8.77 (s, 1H), 8.46 (d, J = 8.1 Hz, 1H), 8.25 (s, 1H), 8.03 - 7.98 (m, 1H), 7.84 (d, J = 8.8 Hz, 1H), 7.57 (dd, J = 8.8, 1.2 Hz, 1H), 6.00 (s, 2H); ¹³C NMR (125 MHz, DMSO-d₆) δ 168.1, 148.3, 145.1, 142.6, 142.3, 136.4, 129.0, 127.5, 126.2, 123.9, 121.7, 121.4, 120.5, 53.3.

### 3. Synthesis of N2-substituted 2H-indazole-6-carboxylic acid hydroxyamide derivatives (Compound 50 to Compound 73, Compound 76)

A mixture of carboxylic acid derivatives (Compound 26 to 49 and Compound 75; 1 mmol), hydroxyamine hydrochloride (3 mmol), benzotriazol-l-yl-oxy-tris-(dimethylamino)phosphonium hexa-fluorophosphate (BOP; 1.7 mmol), and diisopropylethylamine (DIPEA; 3 mmol) was added to DMSO (5-10 ml) and stirred for 6-8 hours at room temperature. The reaction mixture was diluted with water and then extracted with ethyl acetate. The product was purified by column chromatography using chloroform: methanol (90:10) as the eluate.
1) SPA3602 (2-Benzyl-2H-indazole-6-carboxylic acid hydroxyamide) [Compound 50]
   White solid; Yield of 68%, ¹H NMR (500 MHz, DMSO-d₆) δ 11.22 (s, 1H), 9.02 (s, 1H), 8.56 (s, 1H), 8.02 (s, 1H), 7.77 (d, J = 8.7 Hz, 1H), 7.41 (d, J = 8.7 Hz, 1H), 7.39 - 7.29 (m, 5H); ¹³C NMR (125 MHz, DMSO-d₆) δ 165.3, 147.9, 137.2, 130.6, 129.0, 128.4, 124.8, 123.1, 121.3, 120.0, 116.8, 56.9; MS (in mouse) 79.3, (in human) 97.6.
2) SPA3603 (2-(4-Methoxybenzyl)-2H-indazole-6-carboxylic acid hydroxyamide) [Compound 51]
   White solid; Yield of 62%, ¹H NMR (500 MHz, DMSO-d₆) δ 11.22 (s, 1H), 9.01 (s, 1H), 8.49 (s, 1H), 8.02 (s, 1H), 7.75 (d, J = 8.7 Hz, 1H), 7.40 (d, J = 8.7 Hz, 1H), 7.33 (d, J = 8.7 Hz, 2H), 6.92 (d, J = 8.7 Hz, 2H), 5.60 (s, 2H), 3.73 (s, 3H); ¹³C NMR (125 MHz, DMSO-d₆) δ 165.3, 159.5, 14738, 130.5, 130.0, 129.1, 124.4, 123.1, 121.2, 119.9, 11638, 114.4, 56.5, 55,5; MS (in mouse) 98.6, (in human) 76.7.
3) SPA3606 (2-(4-Methylbenzyl)-2H-indazole-6-carboxylic acid hydroxyamide) [Compound 52]
   White solid; Yield of 75%, ¹H NMR (500 MHz, DMSO-d₆) δ 11.22 (s, 1H), 9.01 (s, 1H), 8.51 (s, 1H), 8.02 (s, 1H), 7.76 (d, J = 8.7 Hz, 1H), 7.40 (d, J = 8.7 Hz, 1H), 7.24 (d, J = 8.0 Hz, 2H), 7.17 (d, J = 8.0 Hz, 2H), 5.63 (s, 2H), 2.28 (s, 3H); ¹³C NMR (125 MHz, DMSO-d₆) δ 165.2, 147.8, 137.7, 137.7, 134.1, 130.4, 129.6, 128.4, 124.7, 123.0, 121.2, 119.9, 116.7, 56.7, 21.1.
4) SPA3608 (2-(4-Fluorobenzyl)-2H-indazole-6-carboxylic acid hydroxyamide) [Compound 53]
   White solid; Yield of 66%, ¹H NMR (500 MHz, DMSO-d₆) δ 11.23 (s, 1H), 9.02 (s, 1H), 8.56 (s, 1H), 8.02 (s, 1H), 7.77 (d, J = 8.7 Hz, 1H), 7.39 - 7.43 (m, 3H), 7.20 (t, J = 8.8 Hz, 2H), 5.68 (s, 2H); ¹³C NMR (125 MHz, DMSO-d₆) δ 165.2, 163.2, 161.3, 147.9, 133.4, 130.7, 130.6, 124.8, 121.3, 120.0, 116.8, 115.8, 56.1; MS (in mouse) 71.3, (in human) 85.9.
5) SPA3610 (2-(4-Chlorobenzyl)-2H-indazole-6-carboxylic acid hydroxyamide) [Compound 54]
   White solid; Yield of 77%; ¹H NMR (500 MHz, DMSO-d₆) δ 11.28 (s, 1H), 9.07 (s, 1H), 8.61 (s, 1H), 8.07 (s, 1H), 7.82 (d, J = 8.7 Hz, 1H), 7.45 - 7.49 (m, 3H), 7.40 (d, J = 8.4 Hz, 2H), 5.74 (s, 2H); ¹³C NMR (125 MHz, DMSO-d₆) δ 165.2, 148.0, 136.1, 133.1, 130.7, 130.3, 129.0, 125.0, 123.1, 121.1, 120.1, 116.8, 56.1; MS (in mouse) 40.8, (in human) 49.3.
6) SPA3612 (2-(4-Bromobenzyl)-2H-indazole-6-carboxylic acid hydroxyamide) [Compound 55]
   White solid; Yield of 59%, ¹H NMR (500 MHz, DMSO-d₆) δ 11.23 (s, 1H), 9.02 (s, 1H), 8.56 (s, 1H), 8.02 (s, 1H), 7.77 (d, J = 8.7 Hz, 1H), 7.57 (d, J = 8.4 Hz, 2H), 7.41 (d, J = 8.7 Hz, 1H), 7.28 (d, J = 8.4 Hz, 2H), 5.68 (s, 2H); ¹³C NMR (125 MHz, DMSO-d₆) δ 165.2, 148.0, 136.6, 132.0, 130.7, 130.6, 125.1, 123.0, 121.6, 121.6, 120.1, 116.8, 56.1.
7) SPA3614 (2-(3-Trifluoromethoxybenzyl)-2H-indazole-6-carboxylic acid hydroxyamide) [Compound 56]
   White solid; Yield of 74%, ¹H NMR (500 MHz, DMSO-d₆) δ 11.23 (s, 1H), 9.03 (s, 1H), 8.61 (s, 1H), 8.03 (s, 1H), 7.79 (d, J = 8.7 Hz, 1H), 7.50 (t, J = 7.9 Hz, 1H), 7.42 (d, J = 8.7 Hz, 1H), 7.37 (s, 1H), 7.31 - 7.35 (m, 2H), 5.77 (s, 2H); ¹³C NMR (125 MHz, DMSO-d₆) δ 165.3, 148.7, 148.0, 139.9, 131.1, 130.7, 127.4, 125.2, 123.0, 122.0 121.4, 120.8, 120.1, 119.4, 116.8, 56.1.
8) SPA3616 (2-(4-Trifluoromethoxybenzyl)-2H-indazole-6-carboxylic acid hydroxyamide) [Compound 57]
   White solid; Yield of 71%, ¹H NMR (500 MHz, DMSO-d₆) δ 11.22 (s, 1H), 9.02 (s, 1H), 8.59 (s, 1H), 8.03 (s, 1H), 7.78 (d, J = 8.7 Hz, 1H), 7.46 (d, J = 8.7 Hz, 2H), 7.41 (d, J = 8.7 Hz, 1H), 7.37 (d, J = 8.7 Hz, 2H), 5.74 (s, 2H); ¹³C NMR (125 MHz, DMSO-d₆) δ 165.2, 148.4, 148.0, 136.7, 130.7, 125.1, 123.1, 121.7, 121.3, 120.8, 120.1, 117.0, 116.8, 56.1.
9) SPA3618 (2-Naphthalen-2-ylmethyl-2H-indazole-6-carboxylic acid hydroxyamide) [Compound 58]
   White solid; Yield of 71%, ¹H NMR (500 MHz, DMSO-d₆) δ 11.23 (s, 1H), 9.02 (s, 1H), 8.62 (s, 1H), 8.03 (s, 1H), 7.89 - 7.93 (m, 4H), 7.78 (d, J = 8.7 Hz, 1H), 7.57 - 7.50 (m, 2H), 7.48 (d, J = 7.7 Hz, 1H), 7.42 (d, J = 8.7 Hz, 1H), 5.86 (s, 2H); ¹³C NMR (125 MHz, DMSO-d₆) δ 165.3, 147.9, 134.7, 133.2, 132.9, 130.6, 128.7, 128.2, 127.3, 126.9, 126.3, 125.0, 123.1, 121.3, 120.0, 116.8, 57.1.
10) SPA3620 (2-Phenethyl-2H-indazole-6-carboxylic acid hydroxyamide) [Compound 59]
   White solid; Yield of 77%, ¹H NMR (500 MHz, DMSO-d₆) δ 11.25 (s, 1H), 9.04 (s, 1H), 8.32 (s, 1H), 8.04 (s, 1H), 7.72 (d, J = 8.6 Hz, 1H), 7.38 (d, J = 8.6 Hz, 1H), 7.17-7.27 (m, 5H), 4.72 (t, J = 6.9 Hz, 2H), 3.28 (t, J = 6.9 Hz, 2H); ¹³C NMR (125 MHz, DMSO-d₆) δ 165.3, 147.6, 138.4, 129.0, 128.8, 126.9, 124.7, 122.7, 121.2, 119.8, 116.7, 54.5, 36.4.
11) SPA3622 (2-(2-4-Methoxyphenylethyl)-2H-indazole-6-carboxylic acid hydroxyamide) [Compound 60]
   White solid; Yield of 66%, ¹H NMR (500 MHz, DMSO-d₆) δ 11.25 (s, 1H), 9.04 (s, 1H), 8.31 (s, 1H), 8.04 (s, 1H), 7.72 (d, J = 8.7 Hz, 1H), 7.38 (d, J = 8.7 Hz, 1H), 7.08 (d, J = 8.6 Hz, 2H), 6.81 (d, J = 8.6 Hz, 2H), 4.66 (t, J = 7.2 Hz, 2H), 3.69 (s, 3H), 3.20 (t, J = 7.2 Hz, 2H); ¹³C NMR (125 MHz, DMSO-d₆) δ 165.3, 158.3, 147.6, 130.4, 130.2, 130.1, 124.7, 122.7, 121.2, 119.7, 116.7, 114.2, 55.4, 54.8, 35.6.
12) SPA3624 (2-(3,5-Difluorobenzyl)-2H-indazole-6-carboxylic acid hydroxyamide) [Compound 61]
   White solid; Yield of 79%, ¹H NMR (500 MHz, DMSO-d₆) δ 11.24 (s, 1H), 9.03 (s, 1H), 8.60 (s, 1H), 8.04 (s, 1H), 7.79 (d, J = 8.7 Hz, 1H), 7.42 (d, J = 8.7 Hz, 1H), 7.21 (tt, J = 8.9 Hz, 2.3Hz, 1H), 7.05 (d, J = 6.2 Hz, 2H), 5.73 (s, 2H); ¹³C NMR (125 MHz, DMSO-d₆) δ 165.2, 163.7, 161.8, 148.1, 141.4, 130.8, 125.4, 123.0, 121.4, 120.2, 116.8, 111.5, 103.9, 55.8.
13) SPA3626 (2-(4-Difluoromethoxybenzyl)-2H-indazole-6-carboxylic acid hydroxyamide) [Compound 62]
   White solid; Yield of 62%, ¹H NMR (500 MHz, DMSO-d₆) δ 11.22 (s, 1H), 9.02 (s, 1H), 8.56 (s, 1H), 8.02 (s, 1H), 7.76 (d, J = 8.7, 1H), 7.39 - 7.43 (m, 3H), 7.21 (t, J = 74.0, 1H), 7.17 (d, J = 8.8, 2H), 7.26 - 6.99 (m, 3H), 5.69 (s, 2H); ¹³C NMR (125 MHz, DMSO-d₆) δ 165.2, 151.0, 147.9, 134.1, 130.6, 130.2, 124.9, 123.1, 121.3, 120.0, 119.4, 118.8, 116.8, 56.2.
14) SPA3628 (2-Biphenyl-4-ylmethyl-2H-indazole-6-carboxylic acid hydroxyamide) [Compound 63]
   White solid; Yield of 78%, ¹H NMR (500 MHz, DMSO-d₆) δ 11.23 (s, 1H), 9.02 (s, 1H), 8.60 (s, 1H), 8.04 (s, 1H), 7.79 (d, J = 8.7 Hz, 1H), 7.63 - 7.67 (m, 4H), 7.51 - 7.40 (m, 5H), 7.37 (t, J = 7.3 Hz, 1H), 5.74 (s, 2H); ¹³C NMR (125 MHz, DMSO-d₆) δ 165.2, 147.9, 140.3, 140.1, 136.4, 130.6, 129.4, 129.0, 128.0, 127.3, 127.1, 125.0, 123.1, 121.3, 120.0, 116.8, 56.6.
15) SPA3630 (2-(4-Isopropylbenzyl)-2H-indazole-6-carboxylic acid hydroxyamide) [Compound 64]
   Light yellow solid; Yield of 77%, ¹H NMR (500 MHz, DMSO-d₆) δ 11.22 (s, 1H), 9.01 (s, 1H), 8.54 (s, 1H), 8.02 (s, 1H), 7.76 (d, J = 8.7 Hz, 1H), 7.40 (d, J = 8.7 Hz, 1H), 7.28 (d, J = 8.0 Hz, 2H), 7.23 (d, J = 8.0 Hz, 2H), 5.63 (s, 2H), 2.91 - 2.84 (m, 1H), 1.14 (dd, J = 33.7, 6.8 Hz, 6H).
16) SPA3632 (2-(2-4-Fluorophenylethyl)-2H-indazole-6-carboxylic acid hydroxyamide) [Compound 65]
   Red solid; Yield of 19%, ¹H NMR (500 MHz, DMSO-d₆) δ 11.23 (s, 1H), 9.01 (s, 1H), 8.31 (s, 1H), 8.03 (s, 1H), 7.72 (d, J = 8.7 Hz, 1H), 7.38 (d, J = 8.7 Hz, 1H), 7.20 (dd, J = 8.4, 5.7 Hz, 2H), 7.07 (t, J = 8.4 Hz, 2H), 4.70 (t, J = 7.2 Hz, 2H), 3.27 (t, J = 7.2 Hz, 2H).
17) SPA3634 (2-(3-Fluorobenzyl)-2H-indazole-6-carboxylic acid hydroxyamide) [Compound 66]
   White solid; Yield of 67%, ¹H NMR (500 MHz, DMSO-d₆) δ 11.24 (s, 1H), 9.05 (s, 1H), 8.58 (s, 1H), 8.03 (s, 1H), 7.78 (d, J = 8.7 Hz, 1H), 7.47 - 7.33 (m, 2H), 7.21 - 7.09 (m, 3H), 5.71 (s, 2H); ¹³C NMR (125 MHz, DMSO-d₆) δ 165.3, 161.6, 148.0, 139.9, 131.1, 130.7, 125.2, 124.5, 123.1, 121.4, 120.1, 116.8, 115.3, 115.1, 56.3.
18) SPA3636 (2-(3-Chlorobenzyl)-2H-indazole-6-carboxylic acid hydroxyamide) [Compound 67]
   Light orange solid; Yield of 90%, ¹H NMR (500 MHz, DMSO-d₆) δ 11.22 (s, 1H), 9.01 (s, 1H), 8.59 (s, 1H), 8.02 (s, 1H), 7.78 (d, J = 8.7 Hz, 1H), 7.52 - 7.32 (m, 4H), 7.28 (s, 1H), 5.70 (s, 2H); ¹³C NMR (125 MHz, DMSO-d₆) δ 148.0, 139.6, 133.6, 131.0, 130.8, 128.4, 128.2, 127.1, 125.2, 123.1, 121.4, 120.1, 116.8, 108.3, 56.2.
19) SPA3638 (2-(3-Trifluoromethylbenzyl)-2H-indazole-6-carboxylic acid hydroxyamide) [Compound 68]
   White solid; Yield of 63%, ¹H NMR (500 MHz, DMSO-d₆) δ 11.23 (s, 1H), 9.03 (s, 1H), 8.62 (s, 1H), 8.03 (s, 1H), 7.79 (d, J = 8.8 Hz, 1H), 7.75 (s, 1H), 7.72 - 7.65 (m, 1H), 7.60 (d, J = 5.2 Hz, 2H), 7.42 (d, J = 8.8 Hz, 1H), 5.81 (s, 2H); ¹³C NMR (125 MHz, DMSO-d₆) δ 165.3, 148.1, 138.6, 132.6, 130.8, 130.3, 129.7, 125.3, 125.2, 125.0, 124.5, 123.1, 121.4, 120.2, 116.8, 56.2.
20) SPA3640 (2-(4-Trifluoromethylbenzyl)-2H-indazole-6-carboxylic acid hydroxyamide) [Compound 69]
   White solid; Yield of 62%, ¹H NMR (500 MHz, DMSO-d₆) δ 11.23 (s, 1H), 9.02 (s, 1H), 8.61 (s, 1H), 8.02 (s, 1H), 7.83 - 7.76 (m, 1H), 7.73 (d, J = 8.1 Hz, 2H), 7.49 (d, J = 8.1 Hz, 2H), 7.41 (dd, J = 8.7, 1.4 Hz, 1H), 5.81 (s, 2H); ¹³C NMR (125 MHz, DMSO-d₆) δ 165.3, 148.1, 141.9, 130.8, 129.0, 128.9, 126.0, 125.4, 124.6, 123.1, 121.4, 120.2, 116.8, 56.3.
21) SPA3642 (2-(3-Methoxybenzyl)-2H-indazole-6-carboxylic acid hydroxyamide) [Compound 70]
   White solid; Yield of 77%, ¹H NMR (500 MHz, DMSO-d₆) δ 11.22 (s, 1H), 9.01 (s, 1H), 8.54 (s, 1H), 8.02 (s, 1H), 7.76 (d, J = 8.7 Hz, 1H), 7.43 - 7.35 (m, 1H), 7.26 (t, J = 7.9 Hz, 1H), 6.92 (s, 1H), 6.90 - 6.81 (m, 2H), 5.64 (s, 2H), 3.72 (s, 3H).
22) SPA3644 (2-(3-Benzyloxybenzyl)-2H-indazole-6-carboxylic acid hydroxyamide) [Compound 71]
   White solid; Yield of 54%, ¹H NMR (500 MHz, DMSO-d₆) δ 11.23 (s, 1H), 9.02 (s, 1H), 8.54 (s, 1H), 8.03 (s, 1H), 7.77 (d, J = 8.7 Hz, 1H), 7.46 - 7.23 (m, 7H), 7.00 (s, 1H), 6.97 (dd, J = 8.3, 2.6 Hz, 1H), 6.90 (d, J = 7.6 Hz, 1H), 5.65 (s, 2H), 5.07 (s, 2H); ¹³C NMR (125 MHz, DMSO-d₆) δ 165.3, 158.9, 147.9, 138.7, 137.3, 130.6, 130.2, 128.9, 128.3, 128.2, 125.0, 123.1, 121.3, 120.7, 120.0, 116.9, 115.1, 114.5, 69.7, 56.9.
23) SPA3646 (2-(3,5-Dimethoxybenzyl)-2H-indazole-6-carboxylic acid hydroxyamide) [Compound 72]
   White solid; Yield of 40%, ¹H NMR (500 MHz, DMSO-d₆) δ 11.21 (s, 1H), 9.01 (s, 1H), 8.53 (s, 1H), 8.02 (s, 1H), 7.76 (d, J = 8.8 Hz, 1H), 7.40 (d, J = 8.8 Hz, 1H), 6.65 - 6.31 (m, 3H), 5.59 (s, 2H), 3.70 (s, 6H).
24) SPA3648 (2-(3-Phenoxybenzyl)-2H-indazole-6-carboxylic acid hydroxyamide) [Compound 73]
   White solid; Yield of 67%, ¹H NMR (500 MHz, DMSO-d₆) δ 8.55 (s, 1H), 8.01 (s, 1H), 7.76 (d, J = 8.7 Hz, 1H), 7.46 - 7.28 (m, 4H), 7.14 (t, J = 7.4 Hz, 1H), 7.06 (d, J = 7.7 Hz, 1H), 7.04 - 6.95 (m, 3H), 6.91 (dd, J = 8.2, 2.5 Hz, 1H), 5.68 (s, 2H); ¹³C NMR (125 MHz, DMSO-d₆) δ 157.3, 156.7, 147.9, 139.4, 130.7, 130.7, 130.6, 125.1, 124.1, 123.3, 123.1, 121.4, 120.1, 119.2, 118.4, 118.2, 116.8, 56.5.
25) SPA3650 (2-Pyridin-3-ylmethyl-2H-indazole-6-carboxylic acid hydroxyamide) [Compound 76]

### 4. Synthesis of 2-fluoro-4-methyl-5-nitrobenzoic acid compound (Compound 78)

After completely dissolving 2-fluoro-4-methyl-5-nitrobenzoic acid (1.95 mmol) in H₂SO₄ (3 ml) at 0°C, the mixture of H₂SO₄ (2.34 mmol) and HNO₃ (2.92 mmol) was slowly dropped and reacted for 3 hours. After completion of the reaction, the reaction mixture was placed in ice water, and then the sediment was filtered and dried to obtain nitrobenzoic acid (Compound 78). 1) 2-Fluoro-4-methyl-5-nitrobenzoic acid [Compound 78]

White solid; Yield of 93%, ¹H NMR (500 MHz, DMSO-d₆) δ 13.76 (s, 1H), 8.45 (d, J = 6.8 Hz, 1H), 7.55 (d, J = 11.3 Hz, 1H), 2.59 (s, 3H). ¹³C NMR (125 MHz, DMSO-d₆) δ 163.6, 162.9, 145.1, 141.8, 129.4, 121.7, 118.4, 20.4.

### 5. Synthesis of 2-fluoro-4-methyl-5-nitrobenzoic acid methyl ester compound (Compound 79)

2-Fluoro-4-methyl-5-nitrobenzoic acid (Compound 78, 1.51 mmol) was dissolved in methanol (3 ml), and H₂SO₄(7.53 mmol) was slowly dropped. After stirring the reaction mixture at 65°C for 20 hours, when termination of the reaction was checked, the reaction mixture was placed in ice water, and then the sediment was filtered and dried to obtain 2-fluoro-4-methyl-5-nitrobenzoic acid methyl ester (Compound 79). 1) 2-Fluoro-4-methyl-5-nitrobenzoic acid methyl ester [Compound 79]

White solid; Yield of 89%, ¹H NMR (500 MHz, DMSO-d₆) δ 8.48 (d, J = 6.8 Hz, 1H), 7.61 (d, J = 11.4 Hz, 1H), 3.89 (s, 3H), 2.60 (s, 3H). ¹³C NMR (125 MHz, DMSO-d₆) δ 162.7, 162.7, 145.2, 142.4, 129.2, 121.9, 117.2, 53.3, 20.5.

### 6. Synthesis of 5-amino-2-fluoro-4-methylbenzoic acid methyl ester compound (Compound 80)

2-Fluoro-4-methyl-5-nitrobenzoic acid methyl ester (Compound 79) was completely dissolved in ethyl acetate (5 ml) in the presence of N₂ gas, and then Pd/C was added. After substitution from N₂ gas to Ar gas, stirring was performed at room temperature (rt) for 2 hours, and, once the termination of the reaction was checked, the Pd/C was removed by celite filtration to terminate the reaction. 5-Amino-2-fluoro-4-methylbenzoate methyl ester (Compound 80) was separated by column chromatography using hexane:ethyl acetate (2:1) as an eluate.

1) 5-Amino-2-fluoro-4-methylbenzoic acid methyl ester [Compound 80] Brown solid; Yield of 91%, ¹H NMR (500 MHz, DMSO-d₆) δ 7.10 (d, J = 6.6 Hz, 1H), 6.91 (d, J = 11.7 Hz, 1H), 4.99 (s, 2H), 3.79 (s, 3H), 2.09 (s, 3H). ¹³C NMR (125 MHz, DMSO-d₆) δ 165.0, 153.3, 143.6, 129.6, 118.2, 115.5, 115.3, 52.4, 18.0.

### 7. Synthesis of 5-fluoro-1H-indazole-6-carboxylic acid methyl ester compound [Compound 81]

A mixture of 5-amino-2-fluoro-4-methylbenzoate methyl ester (Compound 80, 0.55 mmol), KOAc (0.28 mmol), and anhydrous acetic acid (1.38 mmol) was stirred in chloroform (5 ml) at room temperature (rt) for 1 hour. Then, isoamyl nitrite (5.51 mmol) was added and reacted by heating at 65°C for 3 hours. After completion of the reaction, the reaction mixture was adjusted to pH 7 with a saturated solution of sodium bicarbonate, and the crude product was extracted with chloroform. 5-Fluoro-1H-indazole-6-carboxylic acid methyl ester (Compound 81) was separated by column chromatography using hexane:ethyl acetate (1:1) as the eluate.

### 1) 5-Fluoro-1H-indazole-6-carboxylic acid methyl ester [Compound 81]

Yellow solid; Yield of 16%, ¹H NMR (500 MHz, CDCl₃) δ 10.30 (s, 1H), 8.17 (d, J = 5.5 Hz, 1H), 8.12 (s, 1H), 7.47 (d, J = 10.6 Hz, 1H), 3.98 (s, 3H). ¹³C NMR (125 MHz, CDCl₃) δ 165.2, 156.4, 136.0, 134.9, 125.6, 119.0, 113.9, 106.7, 52.7.

### 8. Synthesis of N2-substituted 5-fluoro-2H-indazole-6-carboxylic acid methyl ester compounds (Compound 82 to 84)

A mixture of 5-fluoro-1H-indazole-6-carboxylic acid methyl ester (Compound 81; 0.53 mmol), alkyl bromide/chloride (0.58 mmol), and Cs₂CO₃ (0.58 mmol) was reacted in DMF (2 ml) at room temperature (rt) for 2 hours. After completion of the reaction, the reaction mixture was placed in water, and the crude product was extracted with ethyl acetate. N2-substituted 5-fluoro-indazole derivatives (Compounds 82 to 84) were separated by column chromatography using hexane:ethyl acetate (3:1) as eluate, respectively.
1) 2-Benzyl-5-fluoro-2H-indazole-6-carboxylic acid methyl ester [Compound 82]
   Yellow solid; Yield of 55%, ¹H NMR (500 MHz, CDCl₃) δ 8.31 (d, J = 6.3 Hz, 1H), 7.77 (s, 1H), 7.30 - 7.24 (m, 3H), 7.21 - 7.15 (m, 3H), 5.51 (s, 2H), 3.85 (s, 3H). ¹³C NMR (125 MHz, CDCl₃) δ 165.3, 156.3, 145.1, 135.1, 129.1, 128.7, 128.1, 123.9, 123.2, 123.1, 120.0, 104.9, 58.1, 52.4.
2) 2-(3-Chlorobenzyl)-5-fluoro-2H-indazole-6-carboxylic acid methyl ester [Compound 83]
   Yellow solid; Yield of 35%, ¹H NMR (500 MHz, CDCl₃) δ 8.41 (d, J = 6.3 Hz, 1H), 7.91 (s, 1H), 7.35 - 7.26 (m, 4H), 7.16 (dt, J = 6.9, 1.5 Hz, 1H), 5.58 (s, 2H), 3.96 (s, 3H). ¹³C NMR (125 MHz, CDCl₃) δ 165.2, 145.2, 144.2, 136.0, 130.4, 128.9, 128.1, 126.1, 124.0, 123.9, 123.1, 122.0, 120.3, 120.3, 104.6, 57.4, 52.5.
3) 2-(3-Methoxybenzyl)-5-fluoro-2H-indazole-6-carboxylic acid methyl ester [Compound 84]
   Yellow solid; Yield of 32%, ¹H NMR (500 MHz, CDCl₃) δ 8.41 (d, J = 6.2 Hz, 1H), 7.88 (s, 1H), 7.35 - 7.24 (m, 2H), 6.88 (d, J = 8.2 Hz, 2H), 6.82 (s, 1H), 5.58 (s, 2H), 3.96 (s, 3H), 3.77 (s, 3H). ¹³C NMR (125 MHz, CDCl₃) δ 165.3, 160.1, 156.3, 145.0, 136.5, 130.2, 123.89, 123.87, 123.2, 123.0, 120.3, 120.0, 113.9, 104.6, 58.0, 55.3, 52.4.

### 9. Synthesis of N2-substituted 5-fluoro-2H-indazole-6-carboxylic acid compounds (Compounds 85 to 87)

N2-substituted 5-fluoro-2H-indazole-6-carboxylic acid methyl ester (Compound 82 to 84, 0.34 mmol) was dissolved in methanol (1 ml) and THF (1 ml), and 2N NaOH solution (0.5 ml) was slowly added. After stirring the reaction mixture at room temperature for 2 hours, it was placed in water and neutralized with 2N HCl, and then the precipitate was filtered and dried to obtain carboxylic acid derivatives (Compounds 85 to 87).
1) 2-Benzyl-5-fluoro-2H-indazole-6-carboxylic acid [Compound 85]
   Yellow solid; Yield of 76%, ¹H NMR (500 MHz, DMSO-d₆) δ 13.08 (s, 1H), 8.47 (s, 1H), 8.09 (d, J = 6.4 Hz, 1H), 7.48 (d, J = 11.5 Hz, 1H), 7.36 - 7.17 (m, 5H), 5.61 (s, 2H). ¹³C NMR (125 MHz, DMSO-d₆) δ 166.0, 156.7, 144.9, 136.9, 129.1, 128.53, 128.51, 125.3, 122.9, 122.8, 120.8, 105.7, 57.2.
2) 2-(3-Chlorobenzyl)-5-fluoro-2H-indazole-6-carboxylic acid [Compound 86]
   White solid; Yield of 65%, ¹H NMR (500 MHz, MeOD) δ 8.43 - 8.22 (m, 2H), 7.49 – 7.19 (m, 5H), 5.67 (s, 2H).
3) 2-(3-Methoxybenzyl)-5-fluoro-2H-indazole-6-carboxylic acid [Compound 87]
   White solid; Yield of 99%, ¹H NMR (500 MHz, MeOD) δ 8.32 (s, 1H), 8.29 (d, J = 6.3 Hz, 1H), 7.43 (dd, J = 11.2, 1.7 Hz, 1H), 7.27 (t, J = 7.8 Hz, 1H), 6.93 - 6.85 (m, 3H), 5.63 (s, 2H), 3.76 (s, 3H). ¹³C NMR (125 MHz, MeOD) δ 166.4, 160.1, 156.2, 144.8, 137.2, 129.6, 124.6, 123.1, 122.4, 122.4, 119.8, 113.4, 104.6, 57.1, 54.3.

### 10. Synthesis of N2-substituted 2-benzyl-5-fluoro-2H-indazole-6-carboxylic acid compounds (Compounds 88 to 90)

A mixture of carboxylic acid derivatives (Compounds 85 to 87; 0.27 mmol), hydroxyamine hydrochloride (0.82 mmol), benzotriazol-l-yl-oxy-tris-(dimethylamino)phosphonium hexa-fluorophosphate (BOP; 0.41 mmol), and diisopropylethylamine (DIPEA; 0.82 mmol) was added to DMSO (1 to 2 ml) and stirred at room temperature for 6 to 8 hours. The reaction mixture was diluted with water and then extracted with ethyl acetate. The product was purified by column chromatography using chloroform: methanol (90:10) as the eluate.
1) SPA3659 (2-Benzyl-5-fluoro-2H-indazole-6-carboxylic acid hydroxyamide) [Compound 88]
   Brown solid; Yield of 18%, ¹H NMR (500 MHz, DMSO-d₆) δ 11.05 (s, 1H), 9.27 (s, 1H), 8.15 (s, 1H), 7.94 (d, J = 5.3 Hz, 1H), 7.65 (d, J = 10.0 Hz, 1H), 7.37 - 7.17 (m, 5H), 5.71 (s, 2H).
2) SPA3662 (2-(3-chlorobenzyl)-5-fluoro-2H-indazole-6-carboxylic acid hydroxyamide) [Compound 89]
   Light yellow solid; Yield of 24%, ¹H NMR (500 MHz, MeOD) δ 8.37 (s, 1H), 7.92 (d, J = 5.9 Hz, 1H), 7.46 (d, J = 10.6 Hz, 1H), 7.35 - 7.30 (m, 3H), 7.28 - 7.22 (m, 1H), 5.65 (s, 2H). ¹³C NMR (125 MHz, MeOD) δ 163.8, 155.9, 145.1, 138.2, 134.3, 130.1, 128.1, 127.6, 126.0, 124.9, 123.1, 122.2, 119.4, 104.4, 56.3.
3) SPA3663 (2-(3-Methoxybenzyl)-5-fluoro-2H-indazole-6-carboxylic acid hydroxyamide) [Compound 90]
   Brown solid; Yield of 22%, ¹H NMR (500 MHz, MeOD) δ 8.36 (s, 1H), 7.94 (d, J = 5.6 Hz, 1H), 7.48 (d, J = 10.5 Hz, 1H), 7.35 - 7.24 (m, 1H), 6.97 - 6.87 (m, 3H), 5.64 (s, 2H), 3.78 (s, 3H).

### <Experimental Example 1> Evaluation of HDAC6 and HDAC8 inhibitory activity of synthetic compounds

### 1) Deacetylation processes of substrates and development processes

2X HDAC enzymes were dispensed in each well of the reaction plate except for the control well without HDAC enzyme, and buffer was dispensed in the control well without the enzyme. Using acoustic technology (Echo550, nanoliter range), compounds dissolved in 100% DMSO were added to the enzyme mixture, spun down, and pre-cultured at room temperature for 10 minutes. All reaction wells were placed with a 2X substrate mixture (fluorogenic HDAC substrate) to initiate the reaction and then spun down. Culture was performed at 30°C for 1 hour for HDAC6 and 2 hours for HDAC8.

Afterwards, a developer was added along with trichostatin A to halt the reaction and develop fluorescence. Dynamics measurements were performed with Envision (Ex/Em=360/460nm) for 20 minutes with 5-minute intervals, and values from endpoint reading were taken for analysis after the phenomenon reached a plateau.

### 2) HDAC6 and HDAC8 inhibitory activity of synthetic compounds

As a result of examining the inhibitory activity of synthetic compounds on HDAC6 and HDAC8, SPA3602, 3603, 3606, 3608, 3610, 3612, 3614, 3616, 3618, 3620, 3621, 3622, 3624, 3626, 3628, 3630, 3632, 3634, 3636, 3638, 3640, 3642, 3644, 3646, and 3648 showed effective inhibitory activity on HDAC6 with IC₅₀ in the range of 1.3 to 29.3 nM as shown in the following Table, and showed relatively low activity on HDAC8 with IC₅₀ in the range of 500.3 to 8392.0 nM, showing selectivity for HDAC6. From these results, it was found that the N2-substituted derivative is a selective, effective inhibitor on HDAC6 and may be used as a treatment for various diseases such as cancer diseases, inflammatory diseases, autoimmune diseases, and fibrotic diseases.

**TABLE 1**

| Serial No. | Compound No. | SPA No. | Structure | HDAC6 IC₅₀ (nM) | HDAC8 IC₅₀ (nM) |
|---|---|---|---|---|---|
| 1 | 50 | SPA3602 | 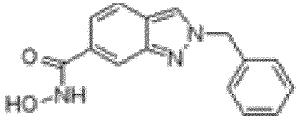 | 2.6 | 1320.0 |
| 2 | 51 | SPA3603 | 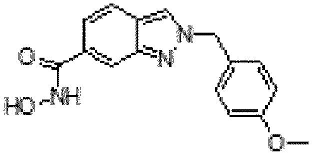 | 5.4 | 765.0 |
| 3 | 52 | SPA3606 | 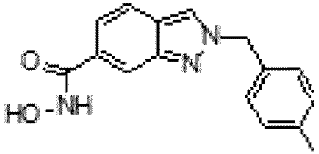 | 7.4 | 699.3 |
| 4 | 53 | SPA3608 | 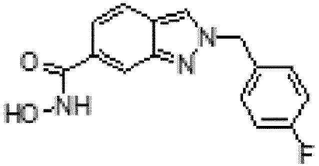 | 5.9 | 932.5 |
| 5 | 54 | SPA3610 | 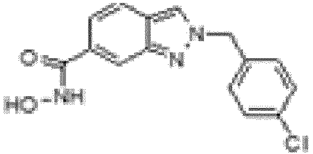 | 2.7 | 775.6 |
| 6 | 55 | SPA3612 | 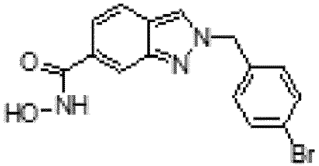 | 3.4 | 592.7 |
| 7 | 56 | SPA3614 | 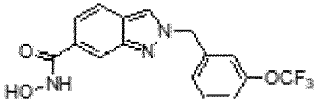 | 4.4 | 1016.0 |
| 8 | 57 | SPA3616 | 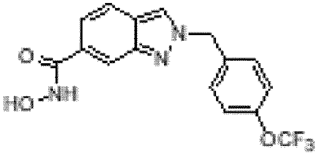 | 5.5 | 1069.0 |
| 9 | 58 | SPA3618 | 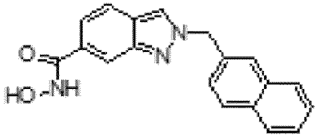 | 4.2 | 870.3 |
| 10 | 59 | SPA3620 | 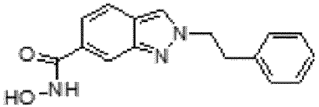 | 4.0 | 1167.0 |
| 11 | 60 | SPA3 622 | 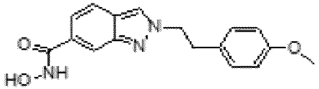 | 4.7 | 1101.0 |
| 12 | 61 | SPA3 624 | 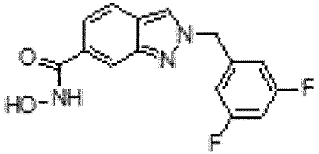 | 3.9 | 877.2 |

**TABLE 2**

| Serial No. | Compound No. | SPA No. | Structure | HDAC6 IC₅₀ (nM) | HDAC8 IC₅₀ (nM) |
|---|---|---|---|---|---|
| 13 | 62 | SPA3626 | 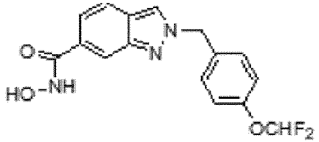 | 4.1 | 500.3 |
| 14 | 63 | SPA3628 | 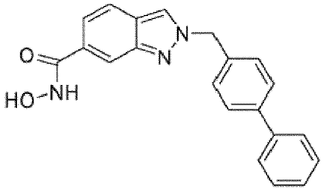 | 3.6 | 1289.0 |
| 15 | 64 | SPA3630 | 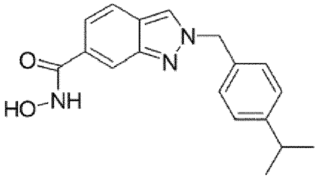 | 29.3 | 2864.0 |
| 16 | 65 | SPA3632 | 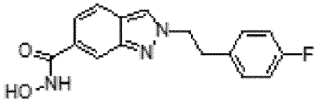 | 6.7 | 1520.0 |
| 17 | 66 | SPA3634 | 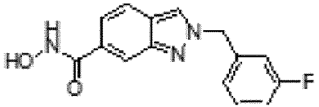 | 1.8 | 1126.0 |
| 18 | 67 | SPA3636 | 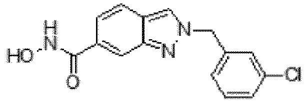 | 1.3 | 8392.0 |
| 19 | 68 | SPA3638 | 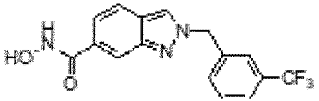 | 3.3 | 738.0 |
| 20 | 69 | SPA3640 | 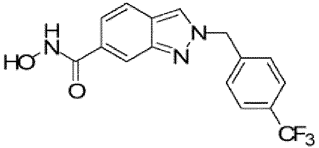 | 3.0 | 598.2 |
| 21 | 70 | SPA3642 | 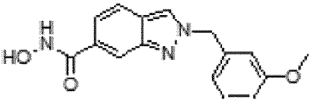 | 1.8 | 966.9 |
| 22 | 71 | SPA3 644 | 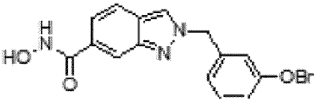 | 13.5 | 2125.0 |
| 23 | 72 | SPA3646 | 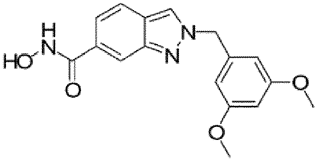 | 3.8 | 814.0 |
| 24 | 73 | SPA3648 | 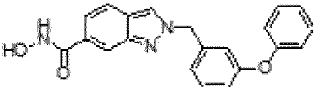 | 22.8 | 2005.0 |
| 25 | 76 | SPA3650 | 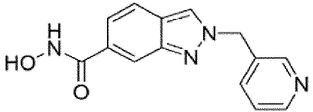 | - | - |
| 26 | 88 | SPA3659 | 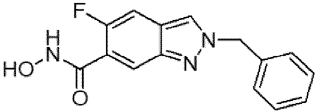 | 74.3 | 3845.0 |
| 27 | 89 | SPA3662 | 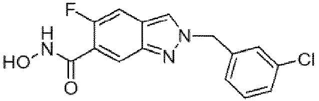 | 30.3 | 2374.0 |
| 28 | 90 | SPA3663 | 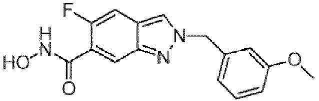 | 75.4 | 2851.0 |

Described below are preparation examples of compositions including Compound 50 (SPA3602) according to the present disclosure, but the present disclosure is merely intended to describe the same in detail, rather than limiting.

<Prescription Example 1> Prescription examples of a pharmaceutical composition

### <Prescription Example 1-1> Preparation of acids

Acids were prepared by mixing 20 mg of Compound 50 (SPA3602), 100 mg of lactose, and 10 mg of talc, and filling the mixture in a gas-tight cloth.

### <Prescription Example 1-2> Preparation of tablets

10 mg of Compound 50 (SPA3602), 100 mg of corn starch, 100 mg of lactose, and 2 mg of magnesium stearate were mixed and then formulated in a tablet form according to the conventional tablet manufacturing method to prepare tablets.

### <Prescription Example 1-3> Preparation of capsules

After mixing 10 mg of Compound 50 (SPA3602), 100 mg of corn starch, 100 mg of lactose, and 2 mg of magnesium stearate, the above ingredients were mixed according to the conventional capsule preparation method and filled into gelatin capsules to prepare capsules.

### <Prescription Example 1-4> Preparation of injections

After mixing 10 mg of Compound 50 (SPA3602), an appropriate amount of sterile distilled water for injection, and an appropriate amount of pH adjuster, preparation was performed in the above ingredient content per 1 ampoule (2 ml) according to the conventional method of injections.

### <Prescription Example 2> Health supplemental food

### <Prescription Example 2-1> Preparation of health supplemental food

After mixing 1 mg of Compound 50 (SPA3602), an appropriate amount of vitamin mixture (70 µg of vitamin A acetate, 1.0 mg of vitamin E, 0.13 mg of vitamin B1, 0.15 mg of vitamin B2, 0.5 mg of vitamin B6, 0.2 µg of vitamin B12, 10 mg of vitamin C, 10 µg of biotin, 1.7 mg of nicotinamide, 50 µg of folic acid, 0.5 mg of calcium pantothenate), and an appropriate amount of mineral mixture (1.75 mg of ferrous sulfate, 0.82 mg of zinc oxide, 25.3 mg of magnesium carbonate, 15 mg of potassium phosphate monobasic, 55 mg of potassium phosphate dibasic, 90 mg of potassium citrate, 100 mg of calcium carbonate, 24.8 mg of magnesium chloride), granules were formed to prepare health food according to the conventional methods.

### <Prescription Example 2-2> Preparation of health drinks

1 mg of Compound 50 (SPA3602), 1000 mg of citric acid, 100 g of oligosaccharide, 2 g of plum concentrate, 1 g of taurine, and purified water were added to adjust a volume in a total of 900 ml, the above ingredients were mixed according to the conventional health drink manufacturing methods and then stirred and heated at 85°C for about 1 hour, and the resulting solution was filtered out in 2 L of sterilized containers, followed by sealing, sterilization, and storage under refrigerated conditions.

While a specific part of the present invention has been described in detail above, it is clear for those skilled in the art that this specific description is merely preferred examples, and the scope of the present invention is not limited thereby. Accordingly, the substantial scope of the present disclosure is defined by the appended claims and their equivalents.

## Claims

1. A compound selected from a compound represented by the following Chemical Formula 1 or a pharmaceutically acceptable salt thereof: 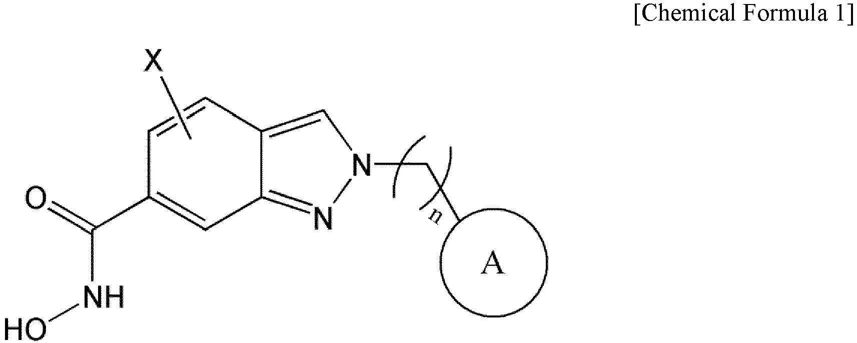 wherein, in the Chemical Formula 1,
X is selected from hydrogen, fluorine, chlorine, or bromine,
A is selected from substituted or unsubstituted phenyl, naphthyl, or a heterocyclic compound,
the substitution is carried out with one or more substituents selected from (C1~C10)alkyl, (C1~C10)alkoxy, halo, difluoromethyl, trifluoromethyl, hydroxy, cyano, nitro, phenyl, benzyloxy, or phenoxy,
the heterocyclic compound is a heteroaryl compound with 5 or 6 rings, and
n is an integer of 1 to 3.

2. The compound of claim 1, wherein the heterocyclic compound is selected from the group consisting of thiophenes, furans, pyrazoles, pyridines, pyrans, oxazines, thiazines, pyrimidines, and piperazines.

3. The compound of claim 1 or claim 2, wherein the compound is one represented by the following Chemical Formula 1-1: 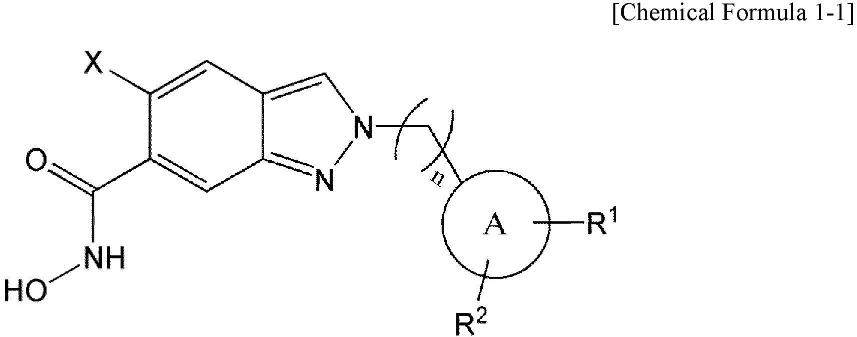 wherein, in the Chemical Formula 1-1,
X is hydrogen or fluorine,
A is selected from phenyl, naphthyl, or pyrimidyl,
R¹ and R² are the same or different and selected from hydrogen, (C1~C4)alkyl, (C1~C4)alkoxy, halo, difluoromethyl, trifluoromethyl, phenyl, benzyloxy, or phenoxy, and
n is an integer of 1 to 3.

4. The compound of claim 3, wherein, in the compound, i) A is selected from phenyl, naphthyl, or pyrimidyl, R¹ is selected from hydrogen, (C1~C4)alkyl, (C1~C4)alkoxy, halo, difluoromethyl, trifluoromethyl, phenyl, benzyloxy, or phenoxy, R² is selected from hydrogen, (C1~C4)alkoxy, or halo, and n is an integer of 1 to 2.

5. The compound of claim 1, wherein the compound is selected from the group consisting of:
2-benzyl-2H-indazole-6-carboxylic acid hydroxyamide (Compound 50);
2-(4-methoxybenzyl)-2H-indazole-6-carboxylic acid hydroxyamide (Compound 51);
2-(4-methylbenzyl)-2H-indazole-6-carboxylic acid hydroxyamide (Compound 52);
2-(4-fluorobenzyl)-2H-indazole-6-carboxylic acid hydroxyamide (Compound 53);
2-(4-chlorobenzyl)-2H-indazole-6-carboxylic acid hydroxyamide (Compound 54);
2-(4-bromobenzyl)-2H-indazole-6-carboxylic acid hydroxyamide (Compound 55);
2-(3-trifluoromethoxybenzyl)-2H-indazole-6-carboxylic acid hydroxyamide (Compound 56);
2-(4-trifluoromethoxybenzyl)-2H-indazole-6-carboxylic acid hydroxyamide (Compound 57);
2-naphthalen-2-ylmethyl-2H-indazole-6-carboxylic acid hydroxyamide (Compound 58);
2-phenethyl-2H-indazole-6-carboxylic acid hydroxyamide (Compound 59);
2-(2-4-methoxyphenylethyl)-2H-indazole-6-carboxylic acid hydroxyamide (Compound 60);
2-(3,5-difluorobenzyl)-2H-indazole-6-carboxylic acid hydroxyamide (Compound 61);
2-(4-difluoromethoxybenzyl)-2H-indazole-6-carboxylic acid hydroxyamide (Compound 62);
2-biphenyl-4-ylmethyl-2H-indazole-6-carboxylic acid hydroxyamide (Compound 63);
2-(4-isopropylbenzyl)-2H-indazole-6-carboxylic acid hydroxyamide (Compound 64);
2-(2-(4-fluorophenyl)ethyl)-2H-indazole-6-carboxylic acid hydroxyamide (Compound 65);
2-(3-fluorobenzyl)-2H-indazole-6-carboxylic acid hydroxyamide (Compound 66);
2-(3-chlorobenzyl)-2H-indazole-6-carboxylic acid hydroxyamide (Compound 67);
2-(3-trifluoromethylbenzyl)-2H-indazole-6-carboxylic acid hydroxyamide (Compound 68);
2-(4-trifluoromethylbenzyl)-2H-indazole-6-carboxylic acid hydroxyamide (Compound 69);
2-(3-methoxybenzyl)-2H-indazole-6-carboxylic acid hydroxyamide (Compound 70);
2-(3-benzyloxybenzyl)-2H-indazole-6-carboxylic acid hydroxyamide (Compound 71);
2-(3,5-dimethoxybenzyl)-2H-indazole-6-carboxylic acid hydroxyamide (Compound 72);
2-(3-phenoxybenzyl)-2H-indazole-6-carboxylic acid hydroxyamide (Compound 73);
2-pyridin-3-ylmethyl-2H-indazole-6-carboxylic acid hydroxyamide (Compound 76);
2-benzyl-5-fluoro-2H-indazole-6-carboxylic acid hydroxyamide (Compound 88);
2-(3-chlorobenzyl)-5-fluoro-2H-indazole-6-carboxylic acid hydroxyamide (Compound 89); and
2-(3-methoxybenzyl)-5-fluoro-2H-indazole-6-carboxylic acid hydroxyamide (Compound 90).

6. The compound of claim 1 or claim 2, wherein the compound selectively inhibits histone deacetylase 6 (HDAC6).

7. A pharmaceutical composition for treating or preventing a histone deacetylase 6 (HDAC6)-associated disease, comprising a compound selected from the compound according to claim 1 or claim 2 or a pharmaceutically acceptable salt thereof.

8. The pharmaceutical composition of claim 7, wherein the compound is one represented by the following Chemical Formula 1-1: 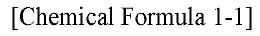 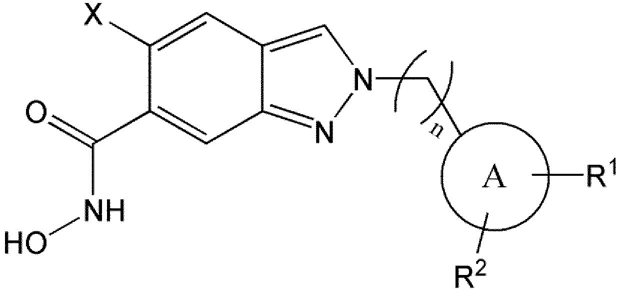 wherein, in the Chemical Formula 1-1,
X is hydrogen or fluorine,
A is selected from phenyl, naphthyl, or pyrimidyl,
R¹ and R² are the same or different and selected from hydrogen, (C1~C4)alkyl, (C1~C4)alkoxy, halo, difluoromethyl, trifluoromethyl, phenyl, benzyloxy, or phenoxy, and
n is an integer of 1 to 3.

9. The pharmaceutical composition of claim 7, wherein, in the compound, i) A is selected from phenyl, naphthyl, or pyrimidyl, R¹ is selected from hydrogen, (C1~C4)alkyl, (C1~C4)alkoxy, halo, difluoromethyl, trifluoromethyl, phenyl, benzyloxy, or phenoxy, R² is selected from hydrogen, (C1~C4)alkoxy, or halo, and n is an integer of 1 to 2.

10. The pharmaceutical composition of claim 7, wherein the compound is selected from the group consisting of:
2-benzyl-2H-indazole-6-carboxylic acid hydroxyamide (Compound 50);
2-(4-methoxybenzyl)-2H-indazole-6-carboxylic acid hydroxyamide (Compound 51);
2-(4-methylbenzyl)-2H-indazole-6-carboxylic acid hydroxyamide (Compound 52);
2-(4-fluorobenzyl)-2H-indazole-6-carboxylic acid hydroxyamide (Compound 53);
2-(4-chlorobenzyl)-2H-indazole-6-carboxylic acid hydroxyamide (Compound 54);
2-(4-bromobenzyl)-2H-indazole-6-carboxylic acid hydroxyamide (Compound 55);
2-(3-trifluoromethoxybenzyl)-2H-indazole-6-carboxylic acid hydroxyamide (Compound 56);
2-(4-trifluoromethoxybenzyl)-2H-indazole-6-carboxylic acid hydroxyamide (Compound 57);
2-naphthalen-2-ylmethyl-2H-indazole-6-carboxylic acid hydroxyamide (Compound 58);
2-phenethyl-2H-indazole-6-carboxylic acid hydroxyamide (Compound 59);
2-(2-4-methoxyphenylethyl)-2H-indazole-6-carboxylic acid hydroxyamide (Compound 60);
2-(3,5-difluorobenzyl)-2H-indazole-6-carboxylic acid hydroxyamide (Compound 61);
2-(4-difluoromethoxybenzyl)-2H-indazole-6-carboxylic acid hydroxyamide (Compound 62);
2-biphenyl-4-ylmethyl-2H-indazole-6-carboxylic acid hydroxyamide (Compound 63);
2-(4-isopropylbenzyl)-2H-indazole-6-carboxylic acid hydroxyamide (Compound 64);
2-(2-(4-fluorophenyl)ethyl)-2H-indazole-6-carboxylic acid hydroxyamide (Compound 65);
2-(3-fluorobenzyl)-2H-indazole-6-carboxylic acid hydroxyamide (Compound 66);
2-(3-chlorobenzyl)-2H-indazole-6-carboxylic acid hydroxyamide (Compound 67);
2-(3-trifluoromethylbenzyl)-2H-indazole-6-carboxylic acid hydroxyamide (Compound 68);
2-(4-trifluoromethylbenzyl)-2H-indazole-6-carboxylic acid hydroxyamide (Compound 69);
2-(3-methoxybenzyl)-2H-indazole-6-carboxylic acid hydroxyamide (Compound 70);
2-(3-benzyloxybenzyl)-2H-indazole-6-carboxylic acid hydroxyamide (Compound 71);
2-(3,5-dimethoxybenzyl)-2H-indazole-6-carboxylic acid hydroxyamide (Compound 72);
2-(3-phenoxybenzyl)-2H-indazole-6-carboxylic acid hydroxyamide (Compound 73);
2-pyridin-3-ylmethyl-2H-indazole-6-carboxylic acid hydroxyamide (Compound 76);
2-benzyl-5-fluoro-2H-indazole-6-carboxylic acid hydroxyamide (Compound 88);
2-(3-chlorobenzyl)-5-fluoro-2H-indazole-6-carboxylic acid hydroxyamide (Compound 89); and
2-(3-methoxybenzyl)-5-fluoro-2H-indazole-6-carboxylic acid hydroxyamide (Compound 90).

11. The pharmaceutical composition of claim 7, wherein the histone deacetylase 6 (HDAC6)-associated disease is selected from the group consisting of cancer diseases, inflammatory diseases, autoimmune diseases, fibrotic diseases, and degenerative diseases.

12. The pharmaceutical composition of claim 11, wherein the cancer diseases are selected from the group consisting of colon cancer, lung cancer, kidney cancer, bladder cancer, liver cancer, thymic cancer, ovarian cancer, cervical cancer, breast cancer, colorectal cancer, stomach cancer, pancreatic cancer, colon cancer, peritoneal metastatic cancer, skin cancer, bladder cancer, prostate cancer, osteosarcoma, fibrous tumor, brain tumor, acute lymphocytic leukemia, acute myeloid leukemia, lymphoma, and neuroblastoma.

13. The pharmaceutical composition of claim 11, wherein the inflammatory diseases or autoimmune diseases are selected from the group consisting of osteoarthritis, rheumatoid arthritis, dermatitis, allergies, atopy, asthma, psoriasis, conjunctivitis, rhinitis, otitis media, sore throat, tonsillitis, pneumonia, gastric ulcer, gastritis, Crohn's disease, inflammatory bowel disease, lupus, hepatitis, cystitis, interstitial cystitis, nephritis, sjogren's syndrome, multiple sclerosis, Hashimoto thyroiditis, polymyositis, scleroderma, Addison disease, vitiligo, pernicious anemia, cystic fibrosis, graft-versus-host disease, graft rejection disease, autoimmune diabetes, diabetic retinopathy, ischemia-reperfusion injury, post-angioplasty restenosis, chronic obstructive pulmonary disease (COPD), Graves disease, and acute and chronic inflammatory diseases.

14. The pharmaceutical composition of claim 11, wherein the fibrotic diseases are selected from the group consisting of pulmonary fibrosis, renal fibrosis, cardiac fibrosis, liver fibrosis, scleroderma, skeletal muscle fibrosis, and diabetic fibrosis.

15. The pharmaceutical composition of claim 11, wherein the degenerative diseases are neurodegenerative diseases selected from the group consisting of cerebral infarction, stroke, memory loss, memory impairment, dementia, forgetfulness, Parkinson's disease, Alzheimer's disease, Pick's disease, Creutzfeldt-Jakob disease, Huntington's disease, and Lou Gehrig's disease.

16. A health functional food for ameliorating or preventing a histone deacetylase 6 (HDAC6)-associated disease, comprising a compound selected from the compound according to claim 1 or claim 2 or a pharmaceutically acceptable salt thereof.

17. A method of treating a histone deacetylase 6 (HDAC6)-associated disease, the method comprising administrating a compound selected from the compound according to claim 1 or claim 2 or a pharmaceutically acceptable salt thereof to a subject suffering from the histone deacetylase 6 (HDAC6)-associated disease.

18. A composition for inhibiting histone deacetylase 6 (HDAC6), comprising a compound selected from the compound according to claim 1 or claim 2 or a pharmaceutically acceptable salt thereof.

19. A method of inhibiting histone deacetylase 6 (HDAC6), the method comprising treating a compound selected from the compound according to claim 1 or claim 2 or a pharmaceutically acceptable salt thereof.
